# EUROPÄISCHE PATENTSCHRIFT

(11) **EP 2 216 027 B1**
(45) Veröffentlichungstag und Bekanntmachung des Hinweises auf die Patenterteilung: **11.05.2016**
(21) Anmeldenummer: 10005374.3
(22) Anmeldetag: 03.07.2003
(51) Int. Cl.: A61K 31/7115, A61K 31/7125, A61K 39/00, A61K 35/66, A61P 37/04

(54) **IMMUNSTIMULATION DURCH CHEMISCH MODIFIZIERTE RNA**
CHEMICALLY MODIFIED RNA FOR IMMUNSTIMULATION
STIMULATION IMMUNITAIRE A L'AIDE DE L'ARN CHIMIQUEMENT MODIFIE

(30) Priorität: 03.07.2002 DE 10229872
(43) Veröffentlichungstag der Anmeldung: 11.08.2010
(62) Teilanmeldung aus: 06007325.1
(73) Patentinhaber: CureVac AG, 72076 Tübingen (DE)
(72) Erfinder: Hoerr, Ingmar, Dr., 70176 Stuttgart (DE); Pascolo, Steve, Dr., 8004 Zürich (CH); Von der Mülbe, Florian, 70563 Stuttgart (DE)
(74) Vertreter: Graf von Stosch, Andreas

(56) Entgegenhaltungen:
- WO-A-01/93902
- WO-A2-01/75164
- WO-A2-02/078614
- US-A- 5 965 720
- GRYAZNOV S M: "Oligonucleotide N3'->P5' phosphoramidates as potential therapeutic agents", BIOCHIMICA ET BIOPHYSICA ACTA. GENE STRUCTURE AND EXPRESSION, ELSEVIER, AMSTERDAM, NL, Bd. 1489, Nr. 1, 10. Dezember 1999 (1999-12-10), Seiten 131-140, XP004275528, ISSN: 0167-4781
- AGRAWAL S: "Antisense oligonucleotides: towards clinical trials", TRENDS IN BIOTECHNOLOGY, ELSEVIER PUBLICATIONS, CAMBRIDGE, GB, Bd. 14, Nr. 10, 1. Oktober 1996 (1996-10-01), Seiten 376-387, XP004035728, ISSN: 0167-7799
- HEIDENREICH O ET AL: "CHEMICALLY MODIFIED RNA: APPROACHES AND APPLICATIONS", FASEB JOURNAL, FED. OF AMERICAN SOC. FOR EXPERIMENTAL BIOLOGY, BETHESDA, MD, US, Bd. 7, Nr. 1, 1993, Seiten 90-96, XP002043382, ISSN: 0892-6638
- MATRAY T J GRYAZNOV S M: "Synthesis and properties of RNA analogs - oligoribonucleotides N3' - p5' phosphoramidites", NUCLEIC ACIDS RESEARCH, OXFORD UNIVERSITY PRESS, SURREY, GB, Bd. 27, Nr. 20, 1999, Seiten 3976-3985, XP002955971, ISSN: 0305-1048
- FIRE A ET AL: "Potent and specific genetic interference by double-stranded RNA in Caenorhabditis elegans", NATURE, NATURE PUBLISHING GROUP, LONDON, GB LNKD- DOI:10.1038/35888, Bd. 391, 19. Februar 1998 (1998-02-19), Seiten 806-811, XP002095876, ISSN: 0028-0836
- SCHEEL B ET AL: "mRNA as immunostimulatory molecule", KREBSIMMUNTHERAPIE ANNUAL MEETING, XX, XX, 9. Mai 2003 (2003-05-09), Seiten 1-13, XP002261545,

## Beschreibung

Die vorliegende Erfindung betrifft eine doppelsträngige Interferenz-RNA zur Verwendung als immunstimulierendes Mittel, die mindestens eine chemische Modifikation aufweist. Des Weiteren betrifft die Erfindung eine Vakzine, die mindestens eine doppelsträngige Interferenz-RNA mit mindestens einer chemischen Modifikation und mindestens ein Antigen enthält. Das erfindungsgemäße immunstimulierende Mittel und die erfindungsgemäße Vakzine werden insbesondere gegen Infektionserkrankungen oder Krebserkrankungen eingesetzt.

RNA spielt in Form von mRNA, tRNA und rRNA bei der Expression der genetischen Information in der Zelle eine zentrale Rolle. Darüber hinaus wurde jedoch in einigen Untersuchungen gezeigt, dass RNA auch als solche an der Regulation von etlichen Prozessen, inbesondere im Organismus von Säugetieren, beteiligt ist. Dabei kann RNA die Rolle als Kommunikationsbotenstoff einnehmen (Benner, FEBS Lett. 1988, 232: 225-228). Des Weiteren wurde eine RNA mit großer Homologie zu einer gewöhnlichen mRNA entdeckt, die aber nicht translatiert wird, sondern eine Funktion in der intrazellulären Regulation ausübt (Brown et al., Cell 1992, 71: 527-542). Derartige regulatorisch wirksame RNA ist durch eine unvollständige Sequenz der Ribosomen-Bindungsstelle (Kozak-Sequenz: GCCGCCACC**AUG**G, wobei das **AUG** das Startcodon bildet (vgl. Kozak, Gene Expr. 1991, 1(2): 117-125) gekennzeichnet, in der sie sich von (normaler) mRNA unterscheidet. Darüber hinaus konnte gezeigt werden, dass diese Klasse der regulatorischen RNA auch in aktivierten Zellen des Immunsystems, bspw. CD4⁺-T-Zellen vorkommt (Liu et al., Genomics 1997, 39: 171-184).

Sowohl bei der klassischen als auch bei der genetischen Vakzinierung tritt häufig das Problem auf, dass nur eine geringe und damit häufig unzureichende Immunantwort im zu therapierenden bzw. zu impfenden Organismus hervorgerufen wird. Daher werden Vakzinen häufig sog. Adjuvanzien, d.h. Substanzen beigefügt, die eine Immunantwort gegen ein Antigen erhöhen und/oder gerichtet beeinflussen können. Im Stand der Technik lange bekannte Adjuvanzien sind z.B. Aluminiumhydroxid, das Freud'sche Adjuvans u.ä. Derartige Adjuvanzien erzeugen jedoch unerwünschte Nebenwirkungen, bspw. sehr schmerzhafte Reizungen und Entzündungen am Ort der Applikation. Des Weiteren werden auch toxische Nebenwirkungen, insbesondere Gewebsnekrosen beobachtet. Schließlich bewirken diese bekannten Adjuvanzien nur eine unzureichende Stimulation der zellulären Immunantwort, da lediglich B-Zellen aktiviert werden.

Des weiteren ist von bakterieller DNA bekannt, dass sie aufgrund des Vorhandenseins von nicht-methylierten CG-Motiven immunstimulierend wirkt, weshalb derartige CpG-DNA als immunstimulierendes Mittel allein und als Adjuvans für Vakzine vorgeschlagen wurde; vgl. US-Patent 5,663,153. Diese immunstimulierende Eigenschaft von DNA kann auch durch DNA-Oligonucleotide erreicht werden, die durch Phosphorthioat-Modifikation stabilisiert sind (US-Patent 6,239,116). Das US-Patent 6,406,705 offenbart schließlich Adjuvans-Zusammensetzungen, welche eine synergistische Kombination aus einem CpG-Oligodesoxyribonucleotid und einem Nicht-Nucleinsäure-Adjuvans enthalten.

Die Verwendung von DNA als immunstimulierendes Mittel oder als Adjuvans in Vakzinen ist jedoch unter mehreren Gesichtspunkten nachteilig. DNA wird in der Blutbahn nur relativ langsam abgebaut, so dass es bei der Verwendung von immunstimulierender DNA zu einer Bildung von Anti-DNA-Antikörpern kommen kann, was im Tiermodell bei der Maus bestätigt wurde (Gilkeson et al., J. Clin. Invest. 1995, 95: 1398-1402). Die mögliche Persistenz der DNA im Organismus kann so zu einer Überaktivierung des Immunsystems führen, welche bekanntermaßen bei Mäusen in einer Splenomegalie resultiert (Montheith et al., Anticancer Drug Res. 1997, 12(5): 421-432). Des Weiteren kann DNA mit dem Wirtsgenom wechselwirken, insbesondere durch Integration in das Wirtsgenom Mutationen hervorrufen. So kann es bspw. zu einer Insertion der eingebrachten DNA in ein intaktes Gen kommen, was eine Mutation darstellt, welche die Funktion des endogenen Gens behindert oder gar vollkommen ausschaltet. Durch solche Integrationsereignisse können einerseits für die Zelle lebenswichtige Enzymsysteme ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidendes Gen verändert wird. Daher kann bei der Verwendung von DNA als immunstimulierendem Mittel ein Risiko der Krebsbildung nicht ausgeschlossen werden.

Riedl et al. (J. Immunol. 2002, 168(10): 4951-4959) offenbaren, dass an eine Arg-reiche Domäne des HBcAg-Nucleocapsids gebundene RNA eine Th1-vermittelte Immunantwort gegen HbcAg hervorruft. Die Arg-reiche Domäne des Nucleocapsids weist Ähnlichkeit zu Protaminen auf und bindet unspezifisch Nucleinsäuren.

Gryaznov, S. M. (Biochemica et Biophysica Acta, 1489(1) (1999): 131-140) berichtet von modifizierten Nukleinsäureanalogen, Oligonukleotid N3' → P5' Phosphoramidaten, enthaltend 3'-Amino anstelle von 3`-Hydroxyryl-Nukleosiden.

Agrawal, S. (Trends in Biotechnology, 14(10) (1996): 376-387) beschreibt Antisense-Oligonukleotide, welche die Fähigkeit zur selektiven Blockierung von krankheitsverursachenden Genen aufweisen und die Produktion von krankheitsassoziierten Proteinen verhindern.

WO 01/93902 offenbart immunologische Zusammensetzungen betreffend immunostimulatorische DNA/RNA-Hybridoligonukleotide sowie Adjuvanssysteme, enthaltend DNA, RNA und/oder RNA/DNA-Hybridoligonukleotide, enthaltend CpG-Dinukleotide.

Modifizierte Oligonukleotide 3-'NHP(O)(O-)O-5' Phosphoramidate, welche eine signifikant erhöhte Resistenz in Hinblick auf den Abbau durch Phosphodiesterase-aufweisen, werden von US 5,965,720 offenbart.

Heidenreich, O. et al. (Faseb Journal, 7(1) (1993): 90-96) berichten von der Verwendung von Phosphorothioat-Internukleotid-Verbindungen unter verschiedenen RNAs und ihren Nutzen für die Identifizierung von Phosphatpositionen, welche für deren Funktion essentiell sind.

Matray, T. J. und Gryaznov, S. M. (Nucleic Acid Research, 27(20) (1999): 3976-3985) beschreiben die Synthese und Charakterisierung von RNA-Mimetika, uniform modifizierten Oligoribonukleotiden N3' → P5' Phophoramidaten, enthaltend alle vier natürlichen Basen sowie Thymidin und 2,6-Diaminopurin.

WO 02/078614 offenbart lrba-Gene, Varianten des lrba-Gens, Fragmenten sowie entsprechenden Polypeptiden, welche durch diese Nukleotide kodiert werden sowie deren Verwendung.

WO 01/75164 berichtet von Gen-spezifischen dsRNA-vermittelten Interferenzen in einem zellfreien System abgeleitet von Drosophila-Embryonen.

Fire, A. et al. (Nature, 391 (1998): 806-811) berichten von wirkungsvollen und spezifischen genetischen Interferenzen doppelsträngiger RNA in *Caenorbabditis elegans.*

Schließlich berichtet Scheel, B. et al. (Krebsimmuntherapie Annual Meeting (2003): 1-13) von Antitumortherapien unter Verwendung von stabilisierter mRNA als immunostimulatorisches Molekül.

Der vorliegenden Erfindung liegt daher die Aufgabe zugrunde, ein neues System zur Verbesserung der Immunstimulation allgemein und der Vakzinierung im Besonderen bereitzustellen, das eine besonders effiziente Immunantwort im zu therapierenden bzw. zu impfenden Patienten hervorruft, die Nachteile bekannter Immunstimulanzien jedoch vermeidet.

Diese Aufgabe wird durch die in den Ansprüchen gekennzeichneten Ausführungsformen der vorliegenden Erfindung gelöst.

Insbesondere wird erfindungsgemäß eine doppelsträngige Interferenz-RNA von 21 Nucleotiden Länge, die mindestens eine chemische Modifikation aufweist, wobei die doppelsträngige Interferenz-RNA durch Ersetzen, Anfügen oder Entfernen einzelner oder mehrerer Atome oder Atomgruppen im Vergleich zu einer natürlich vorkommenden RNA-Spezies verändert ist, zur Verwendung als immunstimulierendes Mittel offenbart. Auch wird gemäß der vorliegenden Erfindung die Verwendung der erfindungsgemäß chemisch modifizierten doppelsträngigen Interferenz-RNA zur Herstellung eines immunstimulierenden Mittels offenbart.

Die vorliegende Erfindung beruht auf der überraschenden Feststellung, dass die als immunstimulierendes Mittel anspruchsgemäß verwendete, chemisch modifizierte doppelsträngige Interferenz-RNA von 21 Nucleotiden Länge Zellen des Immunsystems (vornehmlich Antigen-präsentierende Zellen, insbesondere dentritische Zellen (DC), sowie die Abwehrzellen, bspw. in Form von T-Zellen) besonders stark aktiviert und so das Immunsystem eines Organismus stimuliert. Insbesondere führt die erfindungsgemäß als immunstimulierendes Mittel verwendete, doppelsträngige Interferenz-RNA, enthaltend die chemisch modifizierte RNA, zu einer vermehrten Freisetzung von immunsteuernden Cytokinen, bspw. Interleukinen, wie IL-6, IL-12 usw. Daher ist es bspw. möglich, das erfindungsgemäß bereitgestellte immunstimulierende Mittel gegen Infektionen oder Krebserkrankungen einzusetzen, indem es z.B. in den infizierten Organismus oder den Tumor selbst injiziert wird. Als Beispiele für mit dem erfindungsgemäßen immunstimulierenden Mittel behandelbare Krebserkrankungen können malignes Melanom, Kolon-Karzinom, Lymphome, Sarkome, kleinzelliges Lungenkarzinom, Blastome, usw., genannt werden. Des weiteren wird das immunstimulierende Mittel vorteilhaft gegen Infektionserkrankungen (z.B. virale Infektionskrankheiten, wie AIDS (HIV), Hepatitis A, B oder C, Herpes, Herpes Zoster (Varizellen), Röteln (Rubeola-Virus), Gelbfieber, Dengue usw. (Flavi-Viren), Grippe (Influenza-Viren), hämorrhagische Infektionskrankheiten (Marburg- oder Ebola-Viren), bakterielle Infektionserkrankungen, wie Legionärskrankheit (Legionella), Magengeschwür (Helicobacter), Cholera (Vibrio), *E*.*coli*-Infektionen, Staphylokokken-Infektionen, Salmonellen-Infektionen oder Streptokokken-Infektionen (Tetanus), protozoologische Infektionserkrankungen (Malaria, Schlafkrankheit, Leishmaniose, Toxoplasmose, d.h. Infektionen durch Plasmodium, Trypanosomen, Leishmania und Toxoplasmen, oder Pilzinfektionen, die bspw. durch *Cryptococcur neoformans, Histoplasma capsulatum, Coccidioides immitis, Blastomyces dermatitidis* oder *Candida albicans* hervorgerufen werden) eingesetzt.

Der Begriff "chemische Modifikation" bedeutet, dass die als immunstimulierendes Mittel anspruchsgemäß verwendete, doppelsträngige Interferenz-RNA von 21 Nucleotiden durch Ersetzen, Anfügen oder Entfernen einzelner oder mehrerer Atome oder Atomgruppen im Vergleich zu natürlich vorkommenden RNA-Spezies verändert ist

Vorzugsweise ist die chemische Modifikation derart ausgestaltet, dass die als immunstimulierendes Mittel anspruchsgemäß verwendete, doppelsträngige Interferenz-RNA mindestens ein Analoges natürlich vorkommender Nucleotide enthält.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Besonders bevorzugt ist es, wenn die RNA aus Nucleotidanaloga, bspw. den vorgenannten Analoga, besteht.

Als weitere chemische Modifikationen ist beispielsweise die Anfügung einer sog. "5'-Cap"-Struktur, d.h. ein modifiziertes Guanosin-Nucleotid, zu nennen, insbesondere m7G(5')ppp (5'(A,G(5')ppp(5')A und G(5')ppp(5')G.

Gemäß der vorliegenden Erfindung handelt es sich bei der als immunstimulierendes Mittel anspruchsgemäß verwendeten chemisch modifizierten doppelsträngigen Interferenz-RNA um relativ kurze RNA-Moleküle von 21 Nucleotiden Länge.

Erfindungsgemäß kann die als immunstimulierendes Mittel anspruchsgemäß verwendete doppelsträngige Interferenz-RNA mit einer Länge von 21 Nucleotiden eingesetzt werden, um spezifisch Gene, z.B. von Tumorzellen, auszuschalten, und so diese Zellen gezielt abzutöten oder um darin aktive, für eine maligne Entartung verantwortlich zu machende Gene zu inaktivieren (Elbashir et al., Nature 2001, 411, 494-498).Insbesondere bevorzugt ist es, wenn die RNA-Spezies Phosphorthioat-modifiziert sind.

Gegebenenfalls kann die als immunstimulierendes Mittel anspruchsgemäß verwendete, chemisch modifizierte doppelsträngige Interferenz-RNA von 21 Nucleotiden Länge in Verbindung mit einem pharmazeutisch verträglichen Träger und/oder Vehikel enthalten.

Zur weiteren Erhöhung der Immunogenizität kann das erfindungsgemäße immunstimulierende Mittel ein oder mehrere Adjuvanzien enthalten. Hierbei wird in Bezug auf die Immunstimulation vorzugsweise eine synergistische Wirkung von erfindungsgemäß verwendeter, chemisch modifizierter doppelsträngiger Interferenz-RNA und dem Adjuvans erzielt. Unter "Adjuvans" ist dabei jede Verbindung zu verstehen, die eine Immunantwort begünstigt. In Abhängigkeit der verschiedenen Arten von Adjuvanzien können diesbezüglich verschiedene Mechanismen in Betracht kommen. Bspw. bilden Verbindungen, welche die Reifung der DC erlauben, bspw. Lipopolysaccharide, TNF-α oder CD40-Ligand, eine erste Klasse geeigneter Adjuvanzien. Allgemein kann jedes das Immunsystem beeinflussende Agens von der Art eines "Gefahrsignals" (LPS, GP96 usw.) oder Cytokine, wie GM-CSF, als Adjuvans verwendet werden, welche es erlauben, eine Immunantwort zu verstärken und/oder gerichtet zu beeinflussen. Gegebenenfalls können hierbei auch CpG-Oligonucleotide verwendet werden, wobei allerdings deren unter Umständen auftretenden Nebenwirkungen, wie vorstehend dargelegt, abzuwägen sind. Aufgrund des Vorhandenseins der erfindungsgemäß als immunstimulierendes Mittel verwendeten chemisch modifizierten doppelsträngigen Interferenz-RNA als primärem Immunstimulans ist jedoch nur eine verhältnismäßig geringe Menge an CpG-DNA erforderlich (im Vergleich zu einer Immunstimulation nur mit CpG-DNA), weshalb eine synergistische Wirkung von erfindungsgemäßem immunstimulierendem Mittel und CpG-DNA im allgemeinen zu einer positiven Bewertung dieser Kombination führt. Besonders bevorzugte Adjuvanzien sind Cytokine, wie Monokine, Lymphokine, Interleukine oder Chemokine, z.B. IL-1, IL-2, IL-3, IL-4, IL-5, IL-6, IL-7, IL-8, IL-9, IL-10, IL-12, IFN-α, IFN-γ, GM-CSF, LT-α oder Wachstumsfaktoren, bspw. hGH, bevorzugt. Weitere bekannte Adjuvanzien sind Aluminiumhydroxid, das Freud'sche Adjuvans sowie die weiter unten genannten stabilisierenden kationischen Peptide bzw. Polypeptide, wie Protamin, sowie kationische Polysaccharide, insbesondere Chitosan. Des weiteren sind Lipopeptide, wie Pam3Cys, ebenfalls besonders geeignet, um als Adjuvanzien im immunstimulierenden Mittel der vorliegenden Erfindung eingesetzt zu werden; vgl. Deres et al, Nature 1989, 342: 561-564.

Neben dem direkten Einsatz zum Starten einer Immunreaktion, bspw. gegen einen pathogenen Keim oder gegen einen Tumor, kann das immunstimulierende Mittel auch vorteilhaft zur Verstärkung der Immunantwort gegen ein Antigen eingesetzt werden. Daher kann die erfindungsgemäß als immunstimulierendes Mittel verwendete, chemisch modifizierte, doppelsträngige Interferenz-RNA zur Herstellung einer Vakzine verwendet werden, in der sie als Adjuvans wirkt, das die spezifische Immunantwort gegen das jeweilige Antigen bzw. die jeweiligen Antigene begünstigt.

Somit betrifft ein weiterer Gegenstand der vorliegenden Erfindung eine Vakzine, enthaltend das vorstehend definierte immunstimulierende Mittel und mindestens ein Antigen.

Bei der "klassischen" Vakzinierung enthält die erfindungsgemäße Vakzine bzw. die unter Verwendung der chemisch modifizierten, doppelsträngigen Interferenz-RNA von 21 Nucleotiden Länge, die mindestens eine chemische Modifikation aufweist, herzustellende Vakzine das mindestens eine Antigen selbst. Unter einem "Antigen" ist jede Struktur zu verstehen, welche die Bildung von Antikörpern und/oder die Aktivierung einer zellulären Immunantwort hervorrufen kann. Erfindungemäß werden daher die Begriffe "Antigene" und "Immunogen" synonym verwendet. Beispiele von Antigenen sind Peptide, Polypeptide, also auch Proteine, Zellen, Zellextrakte, Polysaccharide, Polysaccharidkonjugate, Lipide, Plykolipide und Kohlenhydrate. Als Antigene kommen bspw. Tumorantigene, virale, bakterielle, Pilz- und protozoologische Antigene in Betracht. Bevorzugt sind dabei Oberflächenantigene von Tumorzellen und Oberflächenantigene, insbesondere sekretierte Formen, viraler, bakterieller, Pilz- oder protozoologischer Pathogene. Selbstverständlich kann das Antigen in der erfindungsgemäßen Vakzine auch in Form eines an einen geeigneten Träger gekoppelten Haptens vorliegen. Geeignete Träger sind einem Fachmann bekannt und schließen bspw. humanes Serumalbumin (HSA), Polyethylenglykole (PEG) u.ä. ein. Die Kopplung des Haptens an den Träger erfolgt gemäß im Stand der Technik bekannter Verfahren, bspw. im Fall eines Polypeptidträgers über eine Amidbindung an einen Lys-Rest.

Bei der genetischen Vakzinierung mit Hilfe der erfindungsgemäßen Vakzine bzw. der unter Verwendung der anspruchsgemäßen chemisch modifizierten doppelsträngigen Interferenz-RNA herzustellenden genetischen Vakzine erfolgt die Stimulierung einer Immunantwort durch die Einbringung der genetischen Information für das mindestens eine Antigen (in diesem Fall also ein Peptid- oder Polypeptid-Antigen) in Form einer für dieses Antigen codierenden Nucleinsäure, insbesondere einer DNA oder einer RNA (vorzugsweise einer mRNA), in den Organismus bzw. in die Zelle. Die in der Vakzine enthaltene Nucleinsäure wird in das Antigen translatiert, d.h. das von der Nucleinsäure codierte Polypeptid bzw. ein antigenes Peptid wird exprimiert, wodurch eine gegen dieses Antigen gerichtete Immunantwort stimuliert wird. Bei der Vakzinierung gegen einen pathologischen Keim, d.h. ein Virus, ein Bakterium oder ein protozoologischer Keim, wird daher bevorzugt ein Oberflächenantigen eines derartigen Keims zur Vakzinierung mit Hilfe der erfindungsgemäßen Vakzine, enthaltend eine für das Oberflächenantigen codierende Nucleinsäure, verwendet. Im Fall der Verwendung als genetische Vakzine zur Behandlung von Krebs wird die Immunantwort durch Einbringung der genetischen Information für Tumorantigene, insbesondere Proteine, die ausschließlich auf Krebszellen exprimiert werden, erreicht, indem eine erfindungsgemäße Vakzine verabreicht wird, die für ein derartiges Krebsantigen codierende Nucleinsäure enthält. Dadurch wird das bzw. werden die Krebsantigen(e) im Organismus exprimiert, wodurch eine Immunantwort hervorgerufen wird, die wirksam gegen die Krebszellen gerichtet ist.

Die erfindungsgemäße Vakzine kommt insbesondere zur Behandlung von Krebserkrankungen in Betracht. Dabei wird vorzugsweise ein tumorspezifisches Oberflächenantigen (TSSA) oder aber eine Nucleinsäure, die für eine derartiges Antigen codiert, verwendet. So kann die erfindungsgemäße Vakzine zur Behandlung der vorstehend in Bezug auf das anspruchsgemäß bereitgestellte, immunstimulierende Mittel genannten Krebserkrankungen eingesetzt werden. Spezifische Beispiele von erfindungsgemäß in der Vakzine verwendbaren Tumorantigenen sind u.a. 707-AP, AFP, ART-4, BAGE, β-Catenin/m, Bcr-abl, CA-MEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pml/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1.

Des Weiteren wird die erfindungsgemäße Vakzine gegen Infektionserkrankungen, insbesondere die vorstehend in Bezug auf das anspruchsgemäß bereitgestellte, immunstimulierende Mittel genannten Infektionen, eingesetzt. Vorzugsweise werden auch im Falle von Infektionserkrankungen die entsprechenden Oberflächenantigene mit dem stärksten antigenen Potenzial bzw. eine dafür codierende Nucleinsäure in der Vakzine verwendet Bei den genannten Antigenen pathogener Keime bzw. Organismen, insbesondere bei viralen Antigenen, ist dies typischerweise eine sekretierte Form eines Oberflächenantigens. Des weiteren werden erfindungsgemäß bevorzugt Polyepitope bzw. dafür codierende Nucleinsäuren, insbesondere mRNAs eingesetzt, wobei es sich bevorzugt um Polyepitope der vorstehend genannten Antigene, insbesondere Oberflächenantigene von pathogenen Keimen bzw. Organismen oder Tumorzellen, vorzugsweise sekretierter Proteinformen, handelt.

Darüber hinaus kann eine für mindestens ein Antigen codierende Nucleinsäure, die in der erfindungsgemäßen Vakzine enthalten sein kann, neben dem für ein antigenes Peptid bzw. Polypeptid codierenden Abschnitt auch mindestens einen weiteren funktionalen Abschnitt enthalten, der bspw. für ein die Immunantwort förderndes Cytokin, insbesondere die vorstehend unter dem Gesichtspunkt des "Adjuvans" genannten, codiert.

Wie bereits erwähnt; kann es sich bei der mindestens ein Antigen codierenden Nucleinsäure um DNA oder RNA handeln. Zur Einbringung der genetischen Information für ein Antigen in eine Zelle bzw. einen Organismus ist im Falle einer erfindungsgemäßen DNA-Vakzine im allgemeinen ein geeigneter Vektor, welcher einen das jeweilige Antigen codierenden Abschnitt enthält, erforderlich. Als spezifische Beispiele derartiger Vektoren können die Vektoren der Serien pVITRO, pVIVO, pVAC, pBOOST usw. (InvivoGen, San Diego, CA, USA) genannt werden, die unter der URL http://www.invivogen.com beschrieben sind, deren diesbezüglicher Offenbarungsgehalt vollumfänglich in die vorliegende Erfindung aufgenommen ist.

Im Zusammenhang mit erfindungsgemäßen DNA-Vakzinen können verschiedene Verfahren zur Einbringung der DNA in Zellen, wie bspw. Calciumphosphat-Transfektion, Polypren-Transfektion, Protoblasten-Fusion, Elektroporation, Mikroinjektion und Lipofektion, genannt werden, wobei insbesondere die Lipofektion bevorzugt ist.

Bei einer DNA-Vakzine ist jedoch die Verwendung von DNA-Viren als DNA-Vehikel bevorzugt. Derartige Viren haben den Vorteil, dass aufgrund ihrer infektiösen Eigenschaften eine sehr hohe Transfektionsrate zu erzielen ist. Die verwendeten Viren werden genetisch verändert, so dass in der transfizierten Zelle keine funktionsfähigen infektiösen Partikel gebildet werden.

Unter dem Gesichtspunkt der Sicherheit ist der Einsatz von RNA als für mindestens ein Antigen codierende Nucleinsäure in der erfindungsgemäßen Vakzine bevorzugt. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden.

Daher ist es erfindungsgemäß bevorzugt, wenn die für mindestens ein Antigen codierende Nucleinsäure eine mRNA ist, welche einen für das mindestens eine Peptid- oder Polypeptid-Antigen codierenden Abschnitt enthält.

Im Vergleich zu DNA ist RNA in Lösung allerdings wesentlich instabiler. Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Derartige RNase-Verunreinigungen können allgemein nur durch besondere Behandlungen, insbesondere mit Diethylpyrocarbonat (DEPC), beseitigt werden. Der natürliche Abbau von mRNA im Cytoplasma von Zellen ist sehr fein reguliert. Diesbezüglich sind mehrere Mechanismen bekannt. So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Ann. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Daher ist es bevorzugt, sowohl die anspruchsgemäß verwendete, chemische modifizierte doppelsträngige Interferenz-RNA als auch die gegebenenfalls in der Vakzine vorhandene, für ein Antigen codierende RNA, insbesondere eine mRNA, gegenüber dem Abbau durch RNasen zu stabilisieren.

Die Stabilisierung der anspruchsgemäß verwendeten, chemisch modifizierten, doppelsträngigen Interferenz-RNA und gegebenenfalls der für mindestens ein Antigen codierenden mRNA kann dadurch erfolgen, dass die chemisch modifizierte RNA bzw. die gegebenenfalls vorhandene, für das Antigen codierende mRNA mit einer kationischen Verbindung, insbesondere einer polykationischen Verbindung, bspw. einem (poly)kationischen Peptid oder Protein, assoziiert bzw. komplexiert oder daran gebunden ist. Insbesondere ist dabei die Verwendung von Protamin als polykationischem, Nucleinsäure-bindenden Protein besonders wirksam. Des Weiteren ist die Verwendung anderer kationischer Peptide oder Proteine, wie Poly-L-Lysin oder Histonen, ebenfalls möglich. Diese Vorgehensweise zur Stabilisierung der modifizierten mRNA ist in EP-A-1083232 beschrieben, deren diesbezüglicher Offenbarungsgehalt in die vorliegende Erfindung vollumfänglich eingeschlossen ist. Weitere bevorzugte kationische Substanzen, die zur Stabilisierung der anspruchsgemäß verwendeten, chemisch modifizierten, doppelsträngigen Interferenz-RNA und/oder der gegebenenfalls in der erfindungsgemäßen Vakzine enthaltenen mRNA verwendet werden können, schließen kationische Polysaccharide, bspw. Chitosan, ein. Die Assoziierung oder Komplexierung mit kationischen Verbindungen verbessert auch den Transfer der RNA-Moleküle in die zu behandelnden Zellen bzw. den zu behandelnden Organismus.

In den Sequenzen eukaryotischer mRNAs gibt es destabilisierende Sequenzelemente (DSE), an welche Signalproteine binden und den enzymatischen Abbau der mRNA *in vivo* regulieren. Daher werden zur weiteren Stabilisierung der in der erfindungsgemäßen Vakzine enthaltenen mRNA, insbesondere im für das mindestens eine Antigen codierenden Bereich, ein oder mehrere Veränderungen gegenüber dem entsprechenden Bereich der Wildtyp-mRNA vorgenommen, so dass keine destabilisierenden Sequenzelemente enthalten sind. Selbstverständlich ist es erfindungsgemäß ebenfalls bevorzugt, gegebenenfalls in den nicht-translatierten Bereichen (3'- und/oder 5'-UTR) vorhandene DSE aus der mRNA zu eliminieren. Ebenfalls ist es hinsichtlich der erfindungsgemäß als immunstimulierendes Mittel verwendeten, chemisch modifizierten, doppelsträngigen Interferenz-RNA bevorzugt, dass die Sequenz der darin enthaltenen chemisch modifizierten RNA keine derartigen destabilisierenden Sequenzen aufweist.

Beispiele der vorstehenden DSE sind AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674). Die in der erfindungsgemäßen Vakzine enthaltenen RNA-Moleküle sind daher vorzugsweise derart gegenüber der Wildtyp-mRNA verändert, dass sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Vorzugsweise werden auch diese Sequenzmotive aus den chemisch modifizierten RNA-Molekülen der erfindungsgemäß als immunstimulierendes Mittel verwendeten, chemisch modifizierten, doppelsträngigen Interferenz-RNA bzw. aus der gegebenenfalls in der erfindungsgemäßen Vakzine vorhandenen mRNA eliminiert.

Auch die mRNA-Moleküle, welche in der erfindungsgemäßen Vakzine enthalten sein können, weisen bevorzugt eine 5'-Cap-Struktur auf. Als Beispiele von Cap-Strukturen können wiederum m7G(5')ppp (5'(A,G(5')ppp(5')A und G(5')ppp(5')G genannt werden. Weiterhin kann auch die mRNA, wie oben hinsichtlich der anspruchsgemäß verwendeten, chemisch modifizierten, doppelsträngigen Interferenz-RNA erläutert, Analoga natürlich vorkommender Nucleotide aufweisen.

Gemäß einer weiteren bevorzugten Ausführungsform der vorliegenden Erfindung enthält die mRNA einen PolyA-Schwanz von mindestens 50 Nuclotiden, vorzugsweise mindestens 70 Nuclotiden, mehr bevorzugt mindestens 100 Nuclotiden, besonders bevorzugt mindestens 200 Nuclotiden.

Für eine effiziente Translation der für mindestens ein Antigen codierenden mRNA ist weiterhin eine wirksame Bindung der Ribosomen an die Ribosomen-Bindungsstelle (Kozak-Sequenz: GCCGCCACC**AUG**G, das **AUG** bildet das Startcodon) erforderlich. Diesbezüglich ist festgestellt worden, dass ein erhöhter A/U-Gehalt um diese Stelle herum eine effizientere Ribosomen-Bindung an die mRNA ermöglicht.

Des Weiteren ist es möglich, in die für mindestens ein Antigen codierenden mRNA eine oder mehrere sog. IRES (engl. "internal ribosomal entry side") einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische mRNA"). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorligenden Erfindung weist die mRNA in den 5'- und/oder 3'-nicht translatierten Bereichen Stabilisierungssequenzen auf, die befähigt sind, die Halbwertszeit der mRNA im Cytosol zu erhöhen.

Diese Stabifisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis,* genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für α-Globin, α-(I)-Collagen, 15-Lipoxygenase oder für Tyrosin-Hydroxylase codiert (vgl. Holcik et al., Proc. Natl. Acad. Sci. USA 1997, 94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Erhöhung der Translationseffizienz der gegebenenfalls in der erfindungsgemäßen Vakzine enthaltenen mRNA kann der für das mindestens eine Antigen codierende Bereich (sowie jeder weitere gegebenenfalls enthaltene codierende Abschnitt) gegenüber einer entsprechenden Wildtyp-mRNA folgende Modifikationen aufweisen, die entweder alternativ oder in Kombination vorliegen können.

Zum einen kann der G/C-Gehalt des für das Peptid oder Polypeptid codierenden Bereichs der modifizierten mRNA größer sein als der G/C-Gehalt des codierenden Bereichs der für das Peptid oder Polypeptid codierenden Wildtyp-mRNA, wobei die codierte Aminosäuresequenz gegenüber dem Wildtyp unverändert ist.

Diese Modifikation beruht auf der Tatsache, dass für eine effiziente Translation einer mRNA die Sequenzabfolge des zu translatierenden Bereichs der mRNA wesentlich ist. Dabei spielt die Zusammensetzung und die Abfolge der verschiedenen Nucleotide eine große Rolle. Insbesondere sind Sequenzen mit erhöhtem G(Guanosin)/C(Cytosin)-Gehalt stabiler als Sequenzen mit einem erhöhten A(Adenosin)/U(Uracil)-Gehalt. Daher werden erfindungsgemäß unter Beibehaltung der translatierten Aminosäureabfolge die Codons gegenüber der Wildtyp-mRNA derart variiert, dass sie vermehrt G/C-Nucleotide beinhalten. Da mehrere Codons für ein und dieselbe Aminosäure codieren (Degeneration des genetischen Codes), können die für die Stabilität günstigsten Codons ermittelt werden (alternative Codonverwendung, engl. "codon usage").

In Abhängigkeit von der durch die mRNA zu codierenden Aminosäure sind unterschiedliche Möglichkeiten zur Modifikation der mRNA-Sequenz gegenüber der Wildtyp-Sequenz möglich. Im Fall von Aminosäuren, die durch Codons codiert werden, die ausschließlich G- oder C- Nucleotide enthalten, ist keine Modifikation des Codons erforderlich. So erfordern die Codons für Pro (CCC oder CCG), Arg (CGC oder CGG), Ala (GCC oder GCG) und Gly (GGC oder GGG) keine Veränderung, da kein A oder U vorhanden ist.

In folgenden Fällen werden die Codons, welche A-und/oder U-Nucleotide enthalten durch Substituieren anderer Codons, welche die gleichen Aminosäuren codieren, jedoch kein A und/oder U enthalten, verändert. Beispiele sind:
Die Codons für Pro können von CCU oder CCA zu CCC oder CCG verändert werden;
die Codons für Arg können von CGU oder CGA oder AGA oder AGG zu CGC oder CGG verändert werden;
die Codons für Ala können von GCU oder GCA zu GCC oder GCG verändert werden;
die Codons für Gly können von GGU oder GGA zu GGC oder GGG verändert werden.

In anderen Fällen können A bzw. U-Nucleotide zwar nicht aus den Codons eliminiert werden, es ist jedoch möglich, den A- und U-Gehalt durch Verwendung von Codons zu vermindern, die weniger A- und/oder U-Nucleotide enthalten. Zum Beispiel:
Die Codons für Phe können von UUU zu UUC verändert werden;
die Codons für Leu können von UUA, CUU oder CUA zu CUC oder CUG verändert werden;
die Codons für Ser können von UCU oder UCA oder AGU zu UCC, UCG oder AGC verändert werden;
das Codon für Tyr kann von UAU zu UAC verändert werden;
das Stop-Codon UAA kann zu UAG oder UGA verändert werden;
das Codon für Cys kann von UGU zu UGC verändert werden;
das Codon His kann von CAU zu CAC verändert werden;
das Codon für Gln kann von CAA zu CAG verändert werden;
die Codons für Ile können von AUU oder AUA zu AUC verändert werden;
die Codons für Thr können von ACU oder ACA zu ACC oder ACG verändert werden;
das Codon für Asn kann von AAU zu AAC verändert werden;
das Codon für Lys kann von AAA zu AAG verändert werden;
die Codons für Val können von GUU oder GUA zu GUC oder GUG verändert werden;
das Codon für Asp kann von GAU zu GAC verändert werden;
das Codon für Glu kann von GAA zu GAG verändert werden.

Im Falle der Codons für Met (AUG) und Trp (UGG) besteht hingegen keine Möglichkeit der Sequenzmodifikation.

Die vorstehend aufgeführten Substitutionen können selbstverständlich einzeln aber auch in allen möglichen Kombinationen zur Erhöhung des G/C-Gehalts der modifizierten mRNA gegenüber der ursprünglichen Sequenz verwendet werden. So können beispielsweise alle in der ursprünglichen (Wildtyp-) Sequenz auftretenden Codons für Thr zu ACC (oder ACG) verändert werden. Vorzugsweise werden jedoch Kombinationen der vorstehenden Substitutionsmöglichkeiten genutzt, z.B.:
Substitution aller in der ursprünglichen Sequenz für Thr codierenden Codons zu ACC (oder ACG) und Substitution aller ursprünglich für Ser codierenden Codons zu UCC ( oder UCG oder AGC);
Substitution aller in der ursprünglichen Sequenz für Ile codierenden Codons zu AUC und Substitution aller ursprünglich für Lys codierenden Codons zu AAG und Substitution aller ursprünglich für Tyr codierenden Codons zu UAC;
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Arg codierenden Codons zu CGC (oder CGG); Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Gly codierenden Codons zu GGC (oder GGG) und Substituion aller ursprünglich für Asn codierenden Codons zu AAC;
Substitution aller in der ursprünglichen Sequenz für Val codierenden Codons zu GUC (oder GUG) und Substitution aller usprünglich für Phe codierenden Codons zu UUC und Substitution aller ursprünglich für Cys codierenden Codons zu UGC und Substitution aller ursprünglich für Leu codierenden Codons zu CUG (oder CUC) und Substitution aller ursprünglich für Gln codierenden Codons zu CAG und Substitution aller ursprünglich für Pro codierenden Codons zu CCC (oder CCG);
   usw.

Vorzugsweise wird der G/C-Gehalt des für das antigene Peptid bzw. Polypeptid codierenden Bereichs (bzw. jedes anderen gegebenenfalls vorhandenen weiteren Abschnitts) der mRNA um mindestens 7%-Punkte, mehr bevorzugt um mindestens 15%-Punkte, besonders bevorzugt um mindestens 20%-Punkte gegenüber dem G/C-Gehalt des codierten Bereichs der für das entsprechende Peptid bzw. Polypeptid codierenden Wildtyp-mRNA erhöht.

Besonders bevorzugt ist es in diesem Zusammenhang, den G/C-Gehalt der derart modifizierten mRNA, insbesondere im für das mindestens eine antigene Peptid bzw. Polypeptid codierenden Bereich, im Vergleich zur Wildtyp-Sequenz maximal zu erhöhen.

Eine weitere bevorzugte Modifikation einer gegebenenfalls in der vorliegenden Erfindung gekennzeichneten Vakzine enthaltenen mRNA beruht auf der Erkenntnis, dass die Translationseffizienz auch durch eine unterschiedliche Häufigkeit im Vorkommen von tRNAs in Zellen bestimmt wird. Sind daher in einer RNA-Sequenz vermehrt sogenannte "seltene" Codons vorhanden, so wird die entsprechende mRNA deutlich schlechter translatiert als in dem Fall, wenn für relativ "häufige" tRNAs codierende Codons vorhanden sind.

Somit wird erfindungsgemäß in der mRNA (welche in der Vakzine enthalten sein kann), der für das Antigen (d.h. das antigen wirksame Peptid bzw. Polypeptid) codierende Bereich gegenüber dem entsprechenden Bereich der Wildtyp-mRNA derart verändert, dass mindestens ein Codon der Wildtyp-Sequenz, das für eine in der Zelle relativ seltene tRNA codiert, gegen ein Codon ausgetauscht, das für eine in der Zelle relativ häufige tRNA codiert, welche die gleiche Aminosäure trägt wie die relativ seltene tRNA.

Durch diese Modifikation werden die RNA-Sequenzen derart modifiziert, dass Codons eingefügt werden, für die häufig vorkommende tRNAs zur Verfügung stehen.

Welche tRNAs relativ häufig in der Zelle vorkommen und welche demgegenüber relativ selten sind, ist einem Fachmann bekannt; vgl. bspw. Akashi, Curr. Opin. Genet. Dev. 2001, 11(6): 660-666.

Durch diese Modifikation können erfindungsgemäß alle Codons der Wildtyp-Sequenz, die für eine in der Zelle relativ seltene tRNA codieren, jeweils gegen ein Codon ausgetauscht werden, das für eine in der Zelle relativ häufige tRNA codiert, welche jeweils die gleiche Aminosäure trägt wie die relativ seltene tRNA.

Besonders bevorzugt ist es, den in der wie vorstehend beschriebenen mRNA erhöhten, insbesondere maximalen, sequenziellen G/C-Anteil mit den "häufigen" Codons zu verknüpfen, ohne die Aminosäuresequenz des durch den codierenden Bereich der mRNA codierten antigenen Peptids bzw. Polypeptids (ein oder mehrere) zu verändern. Diese bevorzugte Ausführungsform stellt eine besonders effizient translatierte und stabilisierte mRNA für die erfindungsgemäße Vakzine bereit.

Vorzugsweise enthält das erfindungsgemäß bereitgestellte immunstimulierende Mittel neben der anspruchsgemäß verwendeten, chemisch modifizierten doppelsträngigen Interferenz-RNA sowie die erfindungsgemäße Vakzine neben dem anspruchsgemäß bereitgestellten immunstimulierenden Mittel einen pharmazeutisch verträglichen Träger und/oder ein pharmazeutisch verträgliches Vehikel: Entsprechende Wege zur geeigneten Formulierung und Herstellung des erfindungsgemäßen immunstimulierenden Mittels und der Vakzine sind bei "Remingtön's Pharmaceutical Sciences" (Mack Pub. Co., Easton, PA, 1980) offenbart. Für die parenterale Verabreichung kommen als Trägerstoffe bspw. steriles Wasser, sterile Kochsalzlösungen, Polyalkylenglykole, hydrogenierte Naphthalene und insbesondere biokompatible Lactidpolymere, Lactid/Glycolidcopolymer oder Polyoxyethylen-/Polyoxypropylencopolymere in Betracht. Erfindungsgemäß bereitgestellte, immunstimulierende Mittel und Vakzine können Füllsubstanzen oder Substanzen, wie Lactose, Mannitol, Substanzen zur kovalenten Anknüpfung von Polymeren, wie z.B. Polyethylenglykol an erfindungsgemäße antigene Haptene, Peptide oder Polypeptide, Komplexierung mit Metallionen oder Einschluss von Materialien in oder auf besondere Präparationen von Polymerverbindungen, wie z.B. Polylactat, Polyglykolsäure, Hydrogel oder auf Liposomen, Mikroemulsion, Micellen, unilamellare oder multilamellare Vesikel, Erythrozyten-Fragmente oder Sphäroblasten, enthalten. Die jeweiligen Ausführungsformen des anspruchsgemäß bereitgestellten, immunstimulierenden Mittels und der erfindungsgemäßen Vakzine werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wirkstoffkomponenten in der Vakzine bzw. im immunstimulierenden Mittel schließt Formulierungen auf der Basis lipophiler Depots ein (z.B. Fettsäuren, Wachse oder Öle). Im Rahmen der vorliegenden Erfindung werden auch Beschichtungen anspruchsgemäßer immunstimulierender Substanzen und Vakzinsubstanzen oder -zusammensetzungen, enthaltend solche Substanzen, nämlich Beschichtungen mit Polymeren offenbart (z.B. Polyoxamere oder Polyoxamine). Weiterhin können anspruchsgemäße immunstimulierende bzw. Vakzinsubstanzen oder -zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Citrat, HEPES oder Acetat usw. verwendet wird, und der pH typischerweise auf 5,0 bis 8,0, vorzugsweise 6,5 bis 7,5, eingestellt wird. Der Träger bzw. das Vehikel wird zusätzlich vorzugsweise Salzbestandteile enthalten, z.B. Nätriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäß bereitgestellten, immunstimulierenden Mittels und der erfindungsgemäßen Vakzine hängen von der Art der zu bekämpfenden Erkrankung, ggf. deren Stadium, dem Antigen (bei der Vakzine) wie auch dem Körpergewicht, dem Alter und dem Geschlecht des Patienten ab.

Die Konzentration der erfindungsgemäß als immunstimulierendes Mittel verwendeten, chemisch modifizierten, doppelsträngigen Interferenz-RNA wie auch der gegebenenfalls in der erfindungsgemäßen Vakzine enthaltenden codierenden Nucleinsäure in derartigen Formulierungen kann daher innerhalb eines weiten Bereichs von 1 µg bis 100 mg/ml variieren. Das erfindungsgemäß bereitgestellte, immunstimulierende Mittel als auch die erfindungsgemäße Vakzine werden vorzugsweise parenteral, bspw. intravenös, intraarteriell, subkutan, intramuskulär, dem Patienten verabreicht. Ebenso ist es möglich, das immunstimulierende Mittel oder die Vakzine topisch oder oral zu verabreichen.

Somit wird erfindungsgemäß auch die Verwendung zur Prävention und/oder Behandlung der vorstehend genannten Erkrankungen bereitgestellt, welches das Verabreichen des erfindungsgemäß bereitgestellten, immunstimulierenden Mittels oder der erfindungsgemäßen Vakzine an einen Patienten, insbesondere an einen Menschen, umfasst.

Die Figuren zeigen:

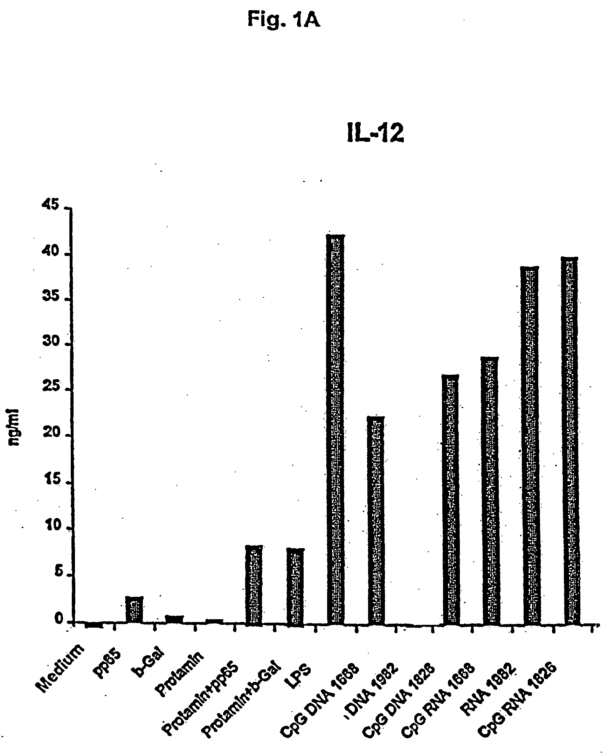

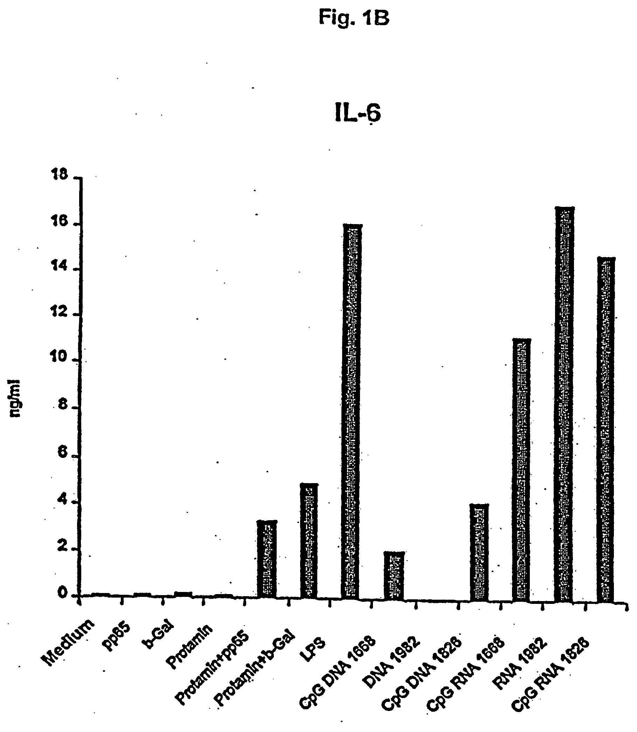

Fig. 1 (Vergleichsbeispiel) zeigt Ergebnisse zur Stimulation der Reifung von dendritischen Zellen (DC) der Maus durch chemisch modifizierte RNA im Vergleich mit mRNA, mit Protamin assoziierter mRNA und DNA. DC der Maus wurden mit 10 µg/ml mRNA (pp65 für pp65-mRNA, β-Gal für β-Galaktosidase-mRNA), durch Protamin stabilisierte mRNA (Protamin+pp65, Protamin+β-Gal), DNA (CpG DNA 1668, DNA 1982 und CpG DNA 1826) sowie Phosporthioat-modifizierte RNA (RNA 1668, RNA 1982 und RNA 1826) stimuliert und die DC-Aktivierung durch Messung der Freisetzung von IL-12 (Fig. 1A) und IL-6 (Fig. 1B) mittels Cytokin-ELISA bestimmt. Als Negativkontrollen diente in beiden Versuchsreihe jeweils Medium ohne Nucleinsäureproben sowie Medium mit zugesetztem Protamin. Als Positivvergleich wurde Lipopolysaccharid (LPS) verwendet. Die Oligodesoxyrebonucleotide (ODN) CpG DNA 1668 und CpG DNA 1826 enthalten jeweils ein CpG-Motiv. Von derartigen ODN ist bekannt, dass sie eine Stimulation von DC hervorrufen (vgl. US-Patent 5,663,153). Das ODN DNA 1982 weist die gleiche Sequenz wie CpG DNA 1826 auf, mit der Ausnahme, dass durch einen Austausch von C nach G das CpG-Motiv entfernt wurde. Die durch Phosphorthioat-Modifizierung stabilisierten Oligoribonucleotide CpG RNA 1668, RNA 1982 und CpG RNA 1826 entsprechen in ihrer Sequenz den vorstehend genannten Vergleichs-ODN der jeweiligen Erkennungsziffer. Im Vergleich zu normaler mRNA zeigen die Protamin-stabilisierten mRNA-Spezies nur eine schwache Aktivierung der DC. Eine im Vergleich dazu sehr viel stärkere Interleukin-Freisetzung wird jedoch in beiden Experimenten durch Phosphorthioat-modifizierten Oligoribonucleotide hervorgerufen, deren Werte mit demjenigen der Positivkontrolle (LPS) vergleichbar sind. Im Vergleich zu Protamin-assoziierter mRNA ergibt sich bei der Stimulation durch Phosphorthioat-modifizierte Oligoribonucleotide eine mehr als verdoppelte Freisetzung von IL-12 bzw. IL-6. Diese überraschend starke Interleukin-Freisetzung aufgrund der Oligoribonucleotide ist zudem unabhängig von CpG-Motiven, wie der Vergleich von Phosphorthioat-modifizierter Oligoribonucleotid RNA 1982 mit dem entsprechenden ODN DNA 1982 zeigt. Das ODN DNA 1982 ruft keine Interleukin-Freisetzung bei den DC hervor, während RNA 1982 eine Interleukin-Freisetzung bewirkt, die im Falle von IL-12 mit der Positivkontrolle LPS vergleichbar ist und bei IL-6 diese sogar übersteigt.

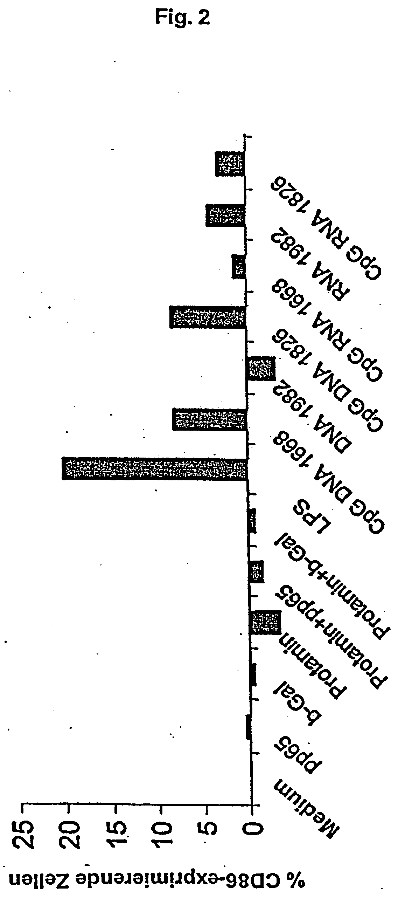

Fig. 2 (Vergleichsbeispiel) zeigt die Ergebnisse der Bestimmung der Expression eines Oberflächenaktivierungsmarkers (CD86) bei DC, die mit den Proben, wie zuvor bei Fig. 1 beschrieben, behandelt wurden. Zur Bestimmung der CD86-Expression wurde 3 Tage nach der Behandlung der DC mit den angegebenen Proben ein Teil der DC mit einem anti-CD86-spezifischen monoklonalen Antikörper markiert und der Prozentanteil der CD86-exprimierenden Zellen mittels Durchflußcytometrie bestimmt. Eine signifikante CD86-Expression wird nur bei den Vergleichs-ODN, die ein CpG-Motiv aufweisen, und Phosphorthioat-modifizierten RNA-Spezies beobachtet. Sämtliche Werte der Nucleinsäurestimulanzien lagen jedoch deutlich unterhalb der Positivkontrolle (LPS). Des weiteren bestätigt die CD86-Bestimmung, dass die durch Phosphorthioat-modifizierte RNA hervorgerufene DC-Aktivierung im Gegensatz zu DNA-Spezies unabhängig von CpG-Motiven ist: Während das CpG-freie ODN DNA 1982 keine CD86-Expression hervorruft, wird beim entsprechenden Phosporthioat-modifizierten Oligoribonucleotid RNA 1982 bei 5% der DC eine CD86-Expression nachgewiesen.

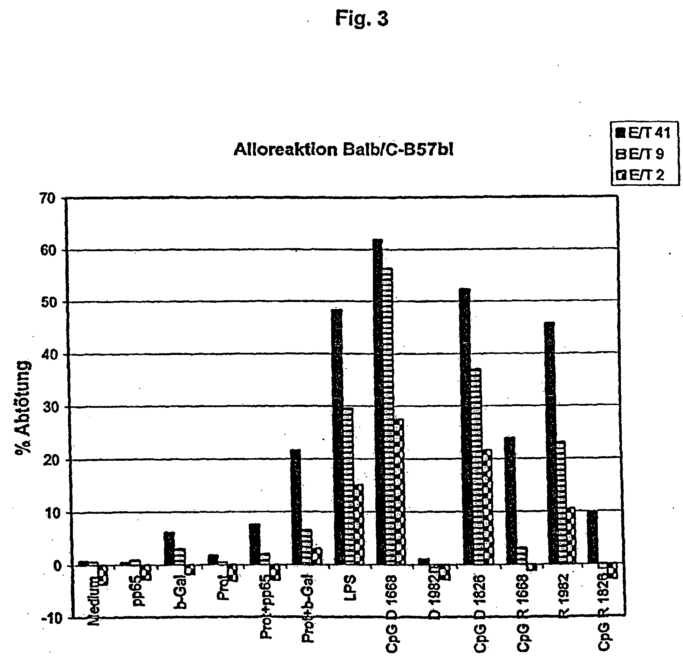

Fig. 3 (Vergleichsbeispiel) zeigt die Ergebnisse eines Alloreaktionstest unter Verwendung von DC, die *in vitro* mit den auf der x-Achse angegebenen Proben (vgl. auch Fig. 1) aktiviert wurden. 3 Tage nach der Stimulation wurden die DC zu frischen Milzzellen aus einem allogenen Tier gegeben und sechs Tage später in einem Cytotoxizitätstest verwendet, bei dem die Freisetzung von ⁵¹Cr bei Zielzellen (P 815) im Vergleich zu Kontrollzellen (EL 4) gemessen wurde. Die Ziel- bzw. Kontrollzellen wurden in einer konstanten Menge ausplattiert und dann 4 Stunden mit jeweils drei verschiedenen Verdünnungen der mit den DC cokultivierten Milzzellen (Effektorzellen) inkubiert, so dass sich ein Verhältnis von Effektorzellen (E) zu Zielzellen (bzw. Kontrollzellen) (T) von 41:1, 9:1 bzw. 2:1 ergab. Auf der y-Achse ist die spezifische Abtötung in Prozent angegeben, die wie folgt berechnet wird: [(gemessene freigesetzte Radioaktivität - spontan freigesetzte Radioaktivität) / (maximale Freisetzung von Radioaktivität - spontan freigesetzte Radioaktivität)] x 100. Mit Protamin assoziierter β-Galaktosidase-mRNA stimulierte DC vermögen bei der geringsten Verdünnung nur eine 20%ige spezifische Abtötung von Zielzellen durch die Effektorzellen hervorzurufen. Durch Phosphorthioat-modifiziertes Oligoribonucleotid stimulierte DC bewirken demgegenüber bei der geringsten Verdünnung eine fast 60%ige, also etwa verdreifachte, spezifische Abtötung der Zielzellen durch die Effektorzellen. Dieser Wert ist vergleichbar mit dem der Positivkontrolle (LPS) und einem ein CpG-Motiv enthaltenden Vergleichs-ODN (CpG DNA 1668). Demgegenüber ist ein ODN ohne CpG-Motiv (DNA 1982) inaktiv, was die Ergebnisse aus den vorhergehenden Experimenten gemäß Fig. 1 und Fig. 2 bestätigt. pp65-mRNA (ohne Protamin), β-Galaktosidase-mRNA (ohne Protamin) sowie Protamin und Medium alleine bewirken keine spezifische Abtötung.

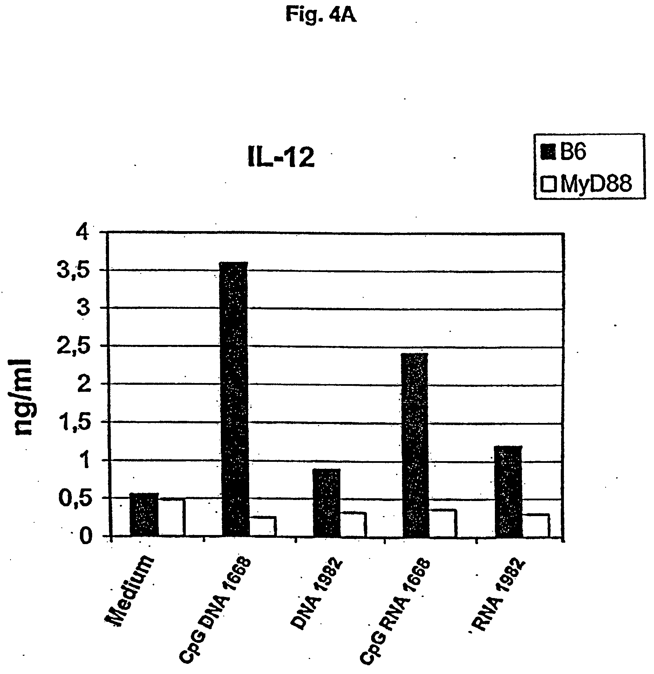

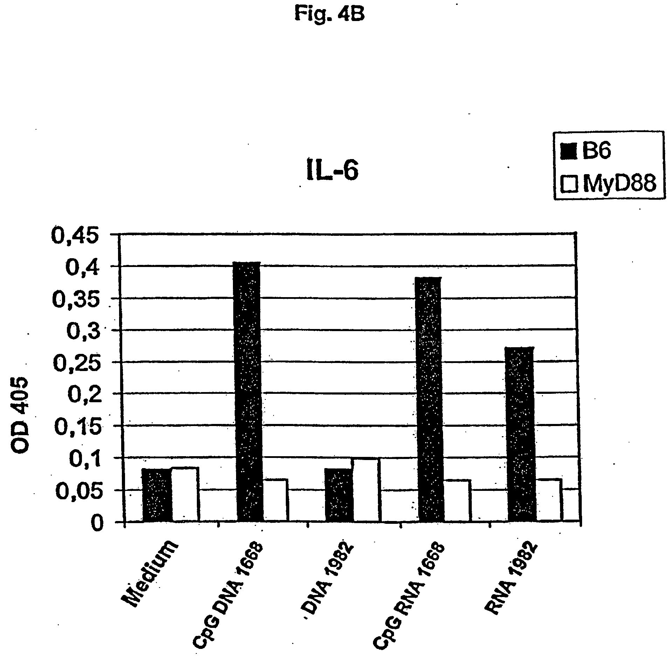

Fig. 4 (Vergleichsbeispiel) zeigt Ergebnisse zur Stimulation der Reifung von dendritischen Zellen (DC) von B6-Mäusen im Vergleich zu MyD88-Knock-Out-Mäusen durch chemisch modifizierte Oligoribonucleotide und Vergleichs-ODN. Als Negativkontrolle diente die Stimulation nur mit Medium. Die Stimulation erfolgte, wie zuvor bei Fig. 1 beschrieben, und die DC-Aktivierung wurde durch Messung der Freisetzung von IL-12 (Fig. 4A) und IL-6 (Fig. 4B) mittels Cytokin-ELISA bestimmt. In Fig. 4A ist auf der y-Achse die IL-12-Konzentration in ng/ml aufgetragen, während in Fig. 4B auf der y-Achse die Absorption bei 405 nm (Absorptionsmaximum des Nachweisreagenz) aufgetragen ist, was der Interleukin-Konzentration direkt proportional ist. In MyD88-Mäusen ist das Protein MyD88, ein Protein aus der Signalkaskade der sog. "toll-like receptors" (TLR), ausgeschaltet. Von TLR-9 ist bspw. bekannt, dass er die Aktivierung von DC durch CpG-DNA vermittelt. DC von B6-Wildtyp-Mäusen werden durch Phosphorthioat-modifizierten Oligoribonucleotide CpG RNA 1688 und RNA 1982 sowie erwartungsgemäß durch das Vergleichs-ODN CpG DNA 1668 aktiviert. Das ODN DNA 1982 (ohne CpG-Motiv) ist wiederum unwirksam. Im Gegensatz dazu kann keine der Proben eine nennenswerte Freisetzung von IL-12 oder Il-6 bei DC aus MyD88-Mäusen hervorrufen. Daher scheint MyD88 zur Aktivierung von DC durch chemisch modifizierten Oligoribonucleotide und durch CpG-ODN erforderlich zu sein.

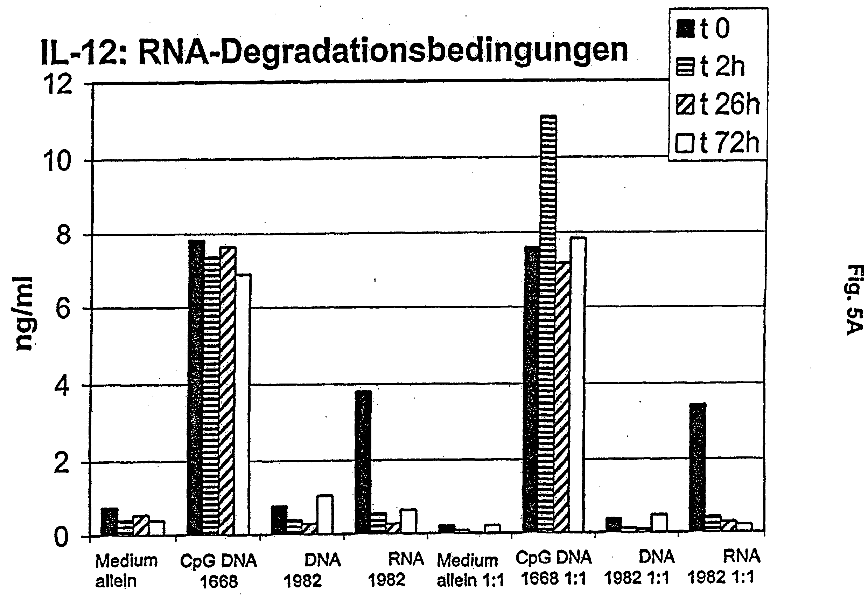

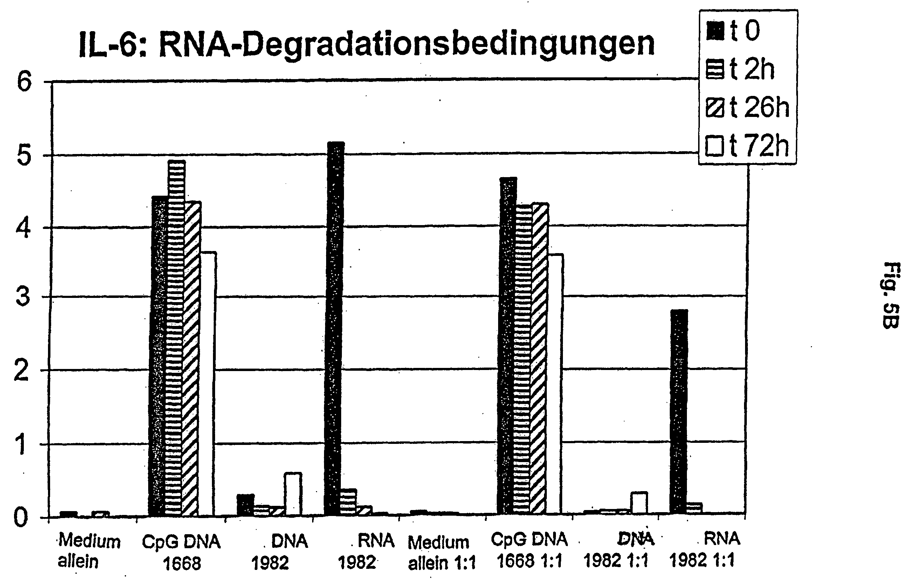

Fig. 5 (Vergleichsbeispiel) zeigt Ergebnisse der Stimulation von DC durch chemisch modifizierte Oligoribonucleotid RNA 1982 und zwei Vergleichs-ODN, die vor dem Einsatz zur DC-Aktivierung für 2, 26 oder 72 h bei 37°C mit Medium inkubiert wurden, das nicht RNase-frei war. Zum Vergleich wurde jeweils eine Probe ohne vorherige Inkubation verwendet (t=0). Die mit "1:1" gekennzeichneten Proben waren gegenüber den jeweils anderen Proben 1:1 mit Puffer verdünnt. Die DC-Aktivierung wurde wiederum durch Bestimmung der Freisetzung von IL-12 (Fig. 5A) und IL-6 (Fig. 5B) mittels Cytokin-ELISA gemessen. Die DC-Aktivierung durch CpG-DNA ist von einer vorherigen Inkubation mit Medium unabhängig. Erwartungsgemäß führt das Vergleichs-ODN ohne CpG-Motiv zu keiner Interleukin-Freisetzung. Beim Oligoribonucleotid RNA 1982 wird ohne Inkubation mit Medium (t=0) eine deutliche Interleukin-Freisetzung gemessen. Bereits nach 2 h Inkubation bei 37°C mit nicht-RNase-freiem Medium wird beim Stimulationsversuch mit Oligoribonucleotid keine nennenswerte Interleukin-Freisetzung mehr beobachtet.

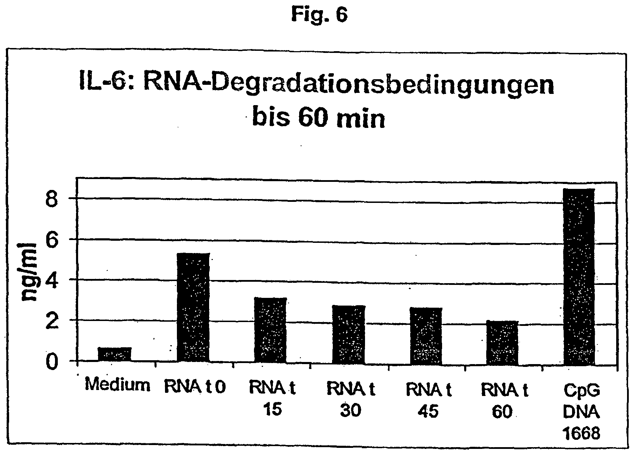

Fig. 6 (Vergleichsbeispiel) zeigt das Ergebnis eines ähnlichen Experiments, wie es in Fig. 5B dargestellt wird, jedoch wurde ein genauerer zeitlicher Verlauf der Auswirkung des RNA-Abbaus auf die DC-Stimulation aufgenommen: Chemisch modifizierte Oligoribonucleotid RNA 1982 wurde wiederum zur Stimulation von DC verwendet und die Aktivierung der DC durch die Messung der IL-6-Freisetzung bestimmt. Vor der Stimulation wurde das Oligoribonucleotid für 15, 30 45 oder 60 min, wie vorstehend bei Fig. 5 beschrieben, mit nicht-RNase-freiem Medium inkubiert. Als Vergleich diente wieder eine nicht mit dem Medium inkubierte Probe (t=0). Als Positivkontrolle wurde das ODN CpG DNA 1668 sowie als Negativkontrolle Medium allein verwendet. Ohne vorherige Inkubation mit nicht-RNase-freiem Medium ergibt sich wieder eine starke DC-Aktivierung durch chemisch modifizierte RNA, wie die IL6-Konzentration von über 5 ng/ml belegt. Dieser Wert sinkt innerhalb einer Stunde Inkubation unter RNA-Degradationsbedingungen auf etwas über 2 ng/ml ab. Dies zeigt, dass die chemisch modifizierte RNA unter physiologischen Bedingungen zwar sehr viel schneller als DNA-Spezies abgebaut wird, die Halbwertszeit jedoch offensichtlich ausreichend lang ist, damit eine immunstimulierende Wirkung entfaltet werden kann.

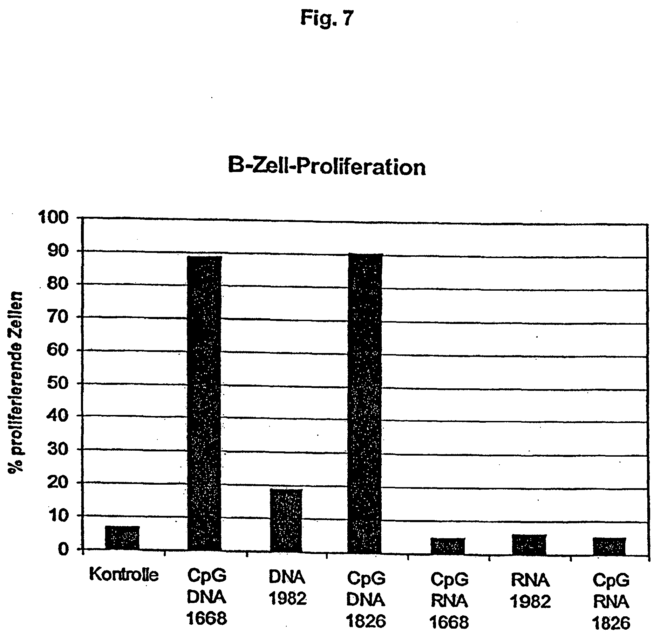

Fig. 7 (Vergleichsbeispiel) zeigt Ergebnisse zur Stimulation der Proliferation von B-Zellen der Maus mit Phosphorthioat-modifizierten Ribonucleotiden (CpG RNA 1668, CpG RNA 1826 und RNA 1982) im Vergleich zu DNA-Spezies (mit CpG-Motiv: CpG DNA 1668 und CpG DNA 1826; ohne CpG-Motiv: DNA 1982). Als Kontrolle diente Medium allein ohne Nucleinsäure-Probe. ODN mit CpG-Motiv führen mit fast 90 % proliferierenden B-Zellen zu einer sehr starken B-Zell-Proliferation. Auch das ODN DNA 1982 (ohne CpG-Motiv), welches sich hinsichtlich der DC-Stimulation als inaktiv erwiesen hat (vgl. Fig. 1 bis 5) rief eine moderate B-Zell-Proliferation (fast 20 % proliferierende Zellen) hervor. Im Gegensatz dazu führte die Stimulation der B-Zellen durch chemisch modifizierte Oligoribonucleotide zu einem Prozentanteil proliferierender B-Zellen, der im Bereich oder sogar unterhalb der Negativkontrolle (in jedem Fall < 10% proliferierende Zellen) liegt.

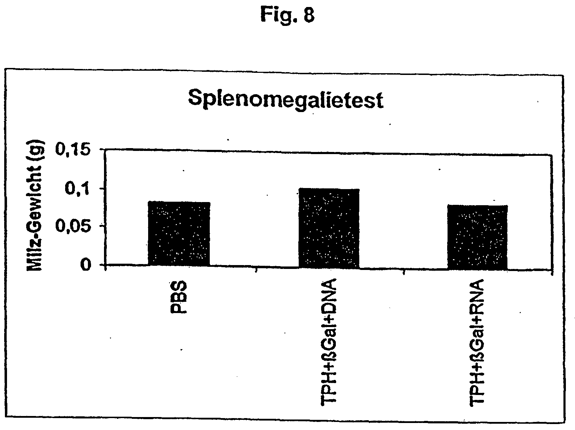

Fig. 8 (Vergleichsbeispiel) zeigt Ergebnisse einer Untersuchung *in vivo* zur Auswirkung von chemisch modifizierter RNA im Vergleich mit DNA auf die Milz von Mäusen. Diesen wurde die jeweilige Nucleinsäure-Spezies zusammen mit einem Antigengemisch (Peptid TPHARGL ("TPH") + β-Galaktosidase ("β-Gal") subkutan injiziert. Nach 10 Tagen wurden die Milzen der Mäuse entnommen und gewogen. Auf der y-Achse ist das Milz-Gewicht in g aufgetragen. Die Balken zeigen jeweils den Mittelwert von zwei unabhängigen Experimenten. Während das Milz-Gewicht bei den mit chemisch modifizierter RNA + Antigengemisch behandelten Mäusen gegenüber der Kontrolle (PBS) mit etwa 0,08 g unverändert ist, zeigt sich bei Mäusen, denen DNA + Antigengemisch injiziert wurde, eine ausgeprägte Splenomegalie, was sich in einem durchschnittlichen Gewicht der Milz von über 0,1 g zeigt.

Die folgenden Beispiele dienen der weiteren Erläuterung.

### Beispiele

Zur Ausführung der nachstehenden Beispiele wurden die folgenden Materialien und Methoden verwendet:

### 1. Zellkultur

Dendritische Zellen (DC) wurden durch Ausspülen des Hinterbeinknochenmarks von BLAB/c-, B6- oder MyD88-Knock-Out-Mäusen mit Medium, Behandlung mit Gey'scher Lösung (zur Lyse der roten Blutzellen) und Filtration durch ein Zellsieb erhalten. Die Zellen wurden dann für 6 Tage in IMDM, enthaltend 10 % Hitze-inaktiviertes fötales Kälberserum (FCS; von PAN), 2 mM L-Glutamin (von Bio Whittaker), 10 mg/ml Streptomycin, 10 U/ml Penicillin (PEN-STREP, von Bio Whittaker) und 51 U/ml GM-CFS (im Folgenden als "Komplettmedium" bezeichnet), in Kulturplatten mit 24 Vertiefungen kultiviert. Nach zwei bzw. vier Tagen wurde das Medium jeweils entfernt und ein äquivalentes Volumen von frischem Medium, welches die vorstehend angegebene Konzentration an GM-CFS enthielt, wurde zugegeben.

### 2. Aktivierung der DC

Nach 6 Tagen wurden die DC in eine Kulturplatte mit 96 Vertiefungen überführt, wobei in jede Vertiefung 200.000 Zellen in 200 µl Komplettmedium gegeben wurden. Die Nucleinsäureproben (DNA, chemisch modifizierte RNA, mRNA oder Protamin-stabilisierte RNA) wurden bei einer Konzentration von 10 µg/ml hinzugefügt.

### 3. RNA-Abbaubedingungen

Es wurden jeweils 5 µl der entsprechenden Nucleinsäureproben (2 µg/µl DNA, nicht-modifizierte RNA oder chemisch modifizierte RNA) in 500 µl Komplettmedium und für 2, 26 oder 72 h bzw. 15, 30, 45 oder 60 min bei 37°C inkubiert. Als Kontrolle diente eine nicht inkubierte Probe (t=0). Anschließend wurden DC mit den Proben, wie unter vorstehendem Punkt 2. beschrieben, stimuliert.

### 4. Cytokin-ELISA

17 Stunden nach der Zugabe der jeweiligen Stimulans wurden 100 µl des Überstands entfernt und 100 µl frisches Medium zugegeben. ELISA-Platten (Nunc Maxisorb) wurden über Nacht mit Fängerantikörpern (Pharmingen) in Bindungspuffer (0,02% NaN₃, 15 mM Na₂CO₃, 15 mM NaHCO₃, pH 9,7) beschichtet. Unspezifische Bindungsstellen wurden mit Phosphat-gepufferter Salzlösung (PBS), enthaltend 1% Rinderserumalbumin (BSA), abgesättigt. Danach wurden jeweils in eine so behandelte Vertiefung 100 µl des jeweiligen Zellkulturüberstands gegeben und 4 Stunden bei 37° C inkubiert. Biotinylierter Antikörper wurde nach 4 Waschschritten mit PBS, enthaltend 0,05% Tween-20, zugegeben. Die Nachweisreaktion wurde durch Zugabe von Streptavidin-gekoppelter Rettichperoxidase (HRP-Streptavidin) und dem Substrat ABTS (Messung der Absorption bei 405 nm) gestartet.

### 5. Durchflußcytometrie

Zur Einfarb-Durchflußcytometrie wurden 2 x 10⁵ Zellen für 20 Minuten bei 4° C in PBS, enthaltend 10% FCS, mit FITC-konjugiertem, monoklonalem anti-CD86-Antikörper (Becton Dickinson) bei geeigneter Konzentration inkubiert. Nach zweimaligem Waschen und Fixierung in 1% Formaldehyd wurden die Zellen mit einem FACScalibur-Durchflußcytometer (Becton Dickinson) und der CellQuestPro-Software analysiert.

### 6. Alloreaktionstest durch ⁵¹Cr-Freisetzung

Milzzellen von B6-Mäusen (C57b16, H-2^{d}-Haplotyp) wurden mit den gemäß vorstehendem Punkt 2. stimulierten DC von BLAB/c-Mäusen (H-2^{d}-Haplotyp) in einem Verhältnis von 1:3 für 5 Tage inkubiert und als Effektorzellen verwendet.

jeweils 5.000 EL-4-Zellen (als Kontrolle) bzw. P815-Zellen (als Zielzellen) wurden in Platten mit 96 Vertiefungen in IMDM mit 10% FCS kultiviert und für eine Stunde mit ⁵¹Cr beladen. Die ⁵¹Cr-markierten Zellen wurden mit den Effektorzellen für 5 Stunden inkubiert (Endvolumen 200 µl). Es wurden jeweils 3 verschiedene Verhältnisse von Effektor- bzw. Kontrollzellen zu Zielzellen (E/T) untersucht: E/T = 41, 9 oder 2. Zur Bestimmung der spezifischen Abtötung wurden 50 µl des Überstands abgenommen und die Radioaktivität unter Verwendung einer Festphasen-Szintillationsplatte (Luma Plate-96, Packard) und einem Szintillationszähler für Microtiterplatten (1450 Microbeta Plus) gemessen. Der Prozentanteil der ⁵¹Cr-Freisetzung wurde anhand der Menge des in das Medium freigesetzten ⁵¹Cr (A) bestimmt und mit der spontanen ⁵¹Cr-Freisetzung von Zielzellen (B) und dem ⁵¹Cr-Gesamtgehalt von Zielzellen (C), die mit 1% Triton-X-100 lysiert wurden, verglichen, wobei sich die spezifische Abtötung aus der folgenden Formel ergibt: % Abtötung = [(A - B)/(C - B)] x 100.

### 7. B-Zell-Proliferationstest

Frische Milzellen einer Maus wurden zweimal mit 10 ml PBS gewaschen und in einer Konzentration von 1 x 10⁷ Zellen/ml in PBS aufgenommen. Es wurde CSFE (FITCmarkiert) in einer Endkonzentration von 500 nM zugegeben und für 3 Minuten inkubiert. Anschließend wurde zweimal mit Medium gewaschen. Es wurde jeweils eine ungefärbte und eine gefärbte Probe im Durchflußcytometer (FACScalibur; Becton Dickinson) untersucht. Zu 200.000 Zellen/Vertiefung einer Kulturplatte mit 96 Vertiefungen (U-förmiger Boden) in 200 µl Medium wurde CpG DNA oder RNA in einer Konzentration von 10 µg/ml gegeben. An Tag 4 nach der Stimulation wurden die Zellen mit B220 CyChrome und CD 69 PE gefärbt und im FACS analysiert.

### 8. In vivo-Untersuchung zur Splenomegalie

50 µg chemisch modifizierte RNA oder Vergleichs-ODN wurden subkutan mit einem Antigengemisch (100 µg Peptid TPHARGL + 100 µg β-Galaktosidase) in jeweils 200 µl PBS in BALB/c-Mäuse (für jede Probe wurden zwei Mäuse verwendet) injiziert. Nach 10 Tagen wurden die Milzen der Mäuse entnommen und gewogen.

### 9. Sequenzen der verwendeten Nucleinsäuren

Oligodesoxyribonucleotide (ODN):
   CpG DNA 1668: 5'-TCCATGACGTTCCTGATGCT-3'
   CpG DNA 1826: 5'-TCCATGACGTTCCTGACGTT-3'
   DNA 1982: 5'-TCCAGGACTTCTCTCAGGIT-3'
Oligoribonucleotide (Phospoxthioat-modifiziert):
   CpG RNA 1668: 5'-UCCAUGACGUUCCUGAUGCU-3'
   CpG RNA 1826: 5'-UCCAUGACGUUCCUGACGUU-3'
   RNA 1982: 5'-UCCAGGACUUCUCUCAGGUU-3'

### Beispiel 1

Um das Vermögen verschiedener Nucleinsäure-Spezies zur Stimulation der Reifung von DC zu bestimmen, wurden DC aus BALB/c-Mäusen gewonnen und mit den unter vorstehendem Punkt 6 angegebenen Oligonucleotiden behandelt. Als weitere Proben wurde jeweils mittels Protamin stabilisierte β-Galaktosidase-mRNA und pp65-RNA verwendet. Mittels ELISA wurde die Freisetzung von IL-12 bzw. IL-6 durch die stimulierten DC bestimmt. Die Stimulierung von DC mittels Protamin-assoziierter mRNA ergab eine schwache Interleukiri-Freisetzung. Im Gegensatz dazu war die durch Phosphorthioat-modifizierte RNA-Spezies hervorgerufene Interleukin-Freisetzung erheblich größer und war sogar mit der Positivkontrolle (Stimulation durch LPS) vergleichbar (Fig. 1A und 1B). Die Vergleichs-ODN, die ein CpG-Motiv enthielten, zeigten eine erwartete Interleukin-Freisetzung durch die DC, wobei jedoch die Interleukin-Freisetzung gegenüber dem Wert, der durch RNA-Spezies entsprechender Sequenz bewirkt wurde, deutlich geringer war (Fig. 1A und 1B).

Um die mittels Cytokin-ELISA demonstrierte Induktion der Reifung der DC zu bestätigen, wurde mittels Durchflußcytometrie die Expression eines für reife DC spezifischen Oberflächenmarkers (CD86) bestimmt. Phosphorthioat-modifizierte RNA-Spezies, nicht jedoch mRNA oder Protamin-assoziierte mRNA, konnten eine deutliche CD86-Expression hervorrufen (Fig. 2).

### Beispiel 2

Des weiteren wurde untersucht, ob die durch die chemisch modifizierten, immunstimulierend wirksamen RNA-Spezies aktivierten DC in der Lage sind, eine Immunantwort in einem allogenen System hervorzurufen (Fig. 3). Dazu wurden Milzzellen der Maus (B6) mit den stimulierten DC aktiviert und als Effektorzellen mit allogenen Zielzellen (P815) zusammengebracht, wobei die Abtötung der Zielzellen mit Hilfe eines ⁵¹Cr-Freisetzungstest bestimmt wurde. Dabei wurden jeweils drei verschiedene Verdünnungen von Effektorzellen mit einer konstanten Anzahl von Zielzellen in Kontakt gebracht. Phosphorthioat-modifizierte RNA ist danach im Vergleich zu Protamin-stabilisierter mRNA sehr viel stärker in der Lage, die Reifung von DC zu aktivierten Zellen hervorzurufen, die eine Immunantwort durch Effektorzellen starten können. Überraschenderweise ist dabei festzustellen, dass durch Phosphorthioat-RNA aktivierte DC eine Immunantwort auslösen können, die ebenso stark ist wie diejenige, die durch ODN, welche CpG-Motive aufweisen, ausgelöst wird.

### Beispiel 3

Es ist bekannt, dass die Aktivierung von DC durch CpG-DN über TLR-9 (engl. "toll-like receptor 9") vermittelt wird (Kaisho et al., Trends Immunol. 2001, 22(2): 78-83). Es wurde daher untersucht, ob auch an der durch chemisch modifizierte, immunstimulierend wirksame RNA bewirkten DC-Aktivierung ebenfalls die TLR-Signalkaskade beteiligt ist. Dazu wurde wiederum anhand der Freisetzung von IL-12 und IL-6 die Aktivierung von DC aus B6-Wildtyp-Mäusen mit der von DC aus B6-Mäusen, denen das Protein MyD88 fehlt, verglichen. MyD88 ist an der TLR-9-Signalkaskade beteiligt. Die starke Freisetzung von IL-12 und IL-6 von DC der B6-Wildtyp-Mäuse bestätigte die Ergebnisse des Beispiels 1 (vgl. Fig. 4A und B, schwarze Balken). Im Gegensatz dazu führte die Stimulation von DC aus MyD88-Knock-Out-Mäusen mit den gleichen Proben zu keiner Aktivierung (vgl. Fig. 4A und B, weiße Balken). Diese Ergebnisse zeigen, dass MyD88 und somit die TLR-9-Signalkaskade sowohl für die CpG-DNA-vermittelte als auch für die durch chemisch modifizierte RNA-vermittelte DC-Aktivierung erforderlich ist.

### Beispiel 4

Um zu untersuchen, ob chemisch modifizierte RNA einem zügigen Abbau unterworfen ist und daher nicht die Gefahr einer Persistenz im Organismus besteht, wurden Oligoribonucleotide unter RNA-Degradationsbedingungen (37°C, unbehandeltes Medium, d.h. nicht-RNase-frei,) für 2, 26 oder 72 h inkubiert und erst danach dem Stimulationstest mit DC zugeführt. Bereits nach einer zweistündigen Inkubation unter RNA-Degradationsbedingungen wurde bei chemisch modifizierter RNA keine Aktivierung der DC mehr beobachtet, wie durch die fehlende IL-12- (Fig. 5A) und IL-6-Freisetzung (Fig. 5B) gezeigt wird. Im Gegensatz dazu hat die vorausgehende Inkubation von CpG-DNA-Spezies keinen Einfluß auf deren Wirksamkeit bei der DC-Aktivierung. Dies zeigt, dass chemisch modifizierte RNA bereits nach relativ kurzer Zeit abgebaut wird, was eine Persistenz im Organismus, die bei DNA auftreten kann, vermeidet.

Chemisch modifizierte RNA wird jedoch nicht so schnell abgebaut, dass sie ihre immunstimulierende Wirkung nicht mehr entfalten kann. Um dies zu belegen, wurde das vorstehende Experiment mit einem Phosphorthioat-modifizierten Oligoribonucleotid (RNA 1982) wiederholt, jedoch die Inkubation unter RNA-Degradationsbedingungen nur für 15, 30, 45 und 60 min durchgeführt. Wie die Freisetzung von IL-6 durch die danach stimulierten DC zeigt, ergibt sich auch nach einer Stunde Inkubation unter RNA-Degradationsbedingungen eine deutliche DC-Aktivierung (Fig. 6).

### Beispiel 5

Die Induktion einer Splenomegalie, die im wesentlichen auf eine starke Aktivierung der B-Zell-Proliferation zurückzuführen ist, stellt ein wesentliches Hindernis der Verwendung von CpG-DNA als immunstimulierendes Adjuvans in Vakzinen dar (vgl. Monteith et al., s.o.). Daher wurde mittels eines B-Zell-Proliferationstest überprüft, ob chemisch modifizierte RNA eine Auswirkung auf die B-Zell-Proliferation hat. Im B-Zell-Proliferationstest wurde ein erwartet hoher Anteil proliferierender Zellen im Fall der Stimulation mit CpG-DNA nachgewiesen. Im Gegensatz dazu war überraschenderweise chemisch modifizierte RNA (unabhängig von etwaig in der Sequenz vorhandener CpG-Motive) diesbezüglich völlig unwirksam (Fig. 7).

Um diese überraschend positive Eigenschaft chemisch modifizierten RNA *in vivo* zu bestätigen, wurde eine Testvakzine, enthaltend ein Phosphorthioat-Oligoribonucleotid (RNA 1982) und ein Antigengemisch aus einem Peptid und β-Galaktosidase, hergestellt und Mäusen subkutan injiziert. Als Vergleich diente eine entsprechende DNA-Testvakzine, die das gleiche Antigengemisch in Verbindung mit einem CpG-ODN (CpG DNA 1826) enthielt. Nach 10 Tagen wurden die Milzen der Mäuse entnommen und gewogen. Verglichen mit der Negativkontrolle (PBS) ergab sich bei mit der DNA-Testvakzine behandelten Mäusen eine signifikante Erhöhung des Milzgewichts. Im Gegensatz dazu wurde bei mit RNA-Testvakzine behandelten Mäusen keine Splenomegalie festgestellt, da hier das Milzgewicht gegenüber der Negativkontrolle unverändert war (Fig. 8). Diese Ergebnisse zeigen, dass bei Verwendung von chemisch modifizierter RNA als immunstimulierendem Mittel oder als Adjuvans in Vakzinen keine im Zusammenhang mit einer unerwünschten B-Zell-Proliferation stehenden Nebenwirkungen auftreten.

Zusammenfassend ist festzustellen, dass chemisch modifizierte RNA die Reifung von DC *in vitro* hervorruft. Die vorstehenden Beispiele belegen, dass chemisch modifizierte RNA, hier in Form kurzer (bspw. 20-merer) synthetischer Oligoribonucleotide (welche Phosphorthioat-modifiziert sind), unreife DC aktiviert und so deren Reifung hervorruft, wie es durch Bestimmung der spezifischen Cytökin-Freisetzung (Fig. 1) und der Expression von Oberflächenaktivierungsmarkern (Fig. 2) gezeigt wird. Die durch chemisch modifizierte RNA hervorgerufene DC-Aktivierung ist deutlich stärker als diejenige, die durch ein Gemisch aus mRNA und der polykationischen Verbindung Protamin, von der bekannt ist, dass sie mit der RNA assoziiert und sie so vor Nucleasen schützt, hervorgerufen wird. Die durch Stimulation mit chemisch modifizierter RNA gereiften DC können eine Immunantwort durch Effektorzellen starten, wie ein Cr⁵¹-Freisetzungstest in einem allogenen System belegt (Fig. 3). Die DC-Aktivierung durch chemisch modifizierte RNA erfolgt vermutlich über eine TLR-vermittelte Signalkaskade (Fig. 4).

Von bakterieller DNA ist bekannt, dass sie aufgrund des Vorhandenseins von nicht-methylierten CG-Motiven immunstimulierend wirkt; vgl. US-Patent 5,663,153. Diese Eigenschaft von DNA kann bei DNA-Oligonucleotiden simuliert werden, die durch Phosphorthioat-Modifikation stabilisiert ist (US-Patent 6,239,116). Von RNA, die durch positiv geladene Proteine komplexiert ist, ist bekannt, dass sie eine immunstimulierende Wirkung aufweist (Riedl et al., 2002, s.o.). Es konnte gezeigt werden, dass RNA, die chemisch modifiziert ist, im Vergleich zu anderer, beispielsweise Protamin-komplexierter, RNA ein sehr viel wirksameres immunstimulierendes Agens ist. Im Gegensatz zu DNA sind bei derartigen chemisch modifizierten RNA-Oligonucleotiden keine CpG-Motive erforderlich. Freie Phosphorthioat-Nucleotide (nicht gezeigt) sind im Gegensatz zu den 20-meren Ribonucleotiden nicht immunstimulierend wirksam.

Chemisch modifizierte immunstimulierende RNA ist der immunstimulierenden DNA jedoch insbesondere dadurch überlegen, dass RNA schneller abgebaut und so aus dem Körper des Patienten entfernt wird, weshalb das Risiko der Persistenz und des Hervorrufens von schweren Nebenwirkungen vermindert bzw. vermieden wird (Fig. 5 und 6). So kann die Verwendung von immunstimulierender DNA als Adjuvans für Vakzine die Bildung von Anti-DNA-Antikörpern hervorrufen, die DNA kann im Organismus persistieren, was bspw. zu einer Überaktivierung des Immunsystems führen kann, welche bekanntermaßen in Mäusen in einer Splenomegalie resultiert (Montheith et al., 1997, s.o.). Die durch DNA-Adjuvanzien hervorgerufene Splenomegalie geht wesentlich auf eine Stimulation der B-Zell-Proliferation zurück, die bei RNA-Adjuvanzien nicht auftritt (Fig. 7 und 8). Des Weiteren kann DNA mit dem Wirtsgenom wechselwirken, insbesondere durch Integration in das Wirtsgenom Mutationen hervorrufen. All diese hohen Risiken können bei Verwendung chemisch modifizierter RNA zur Herstellung von immunstimulierenden Mitteln oder Vakzinen, insbesondere zur Impfung gegen oder zur Behandlung von Krebs- oder Infektionserkrankungen, bei besserer oder vergleichbarer Immunstimulation vermieden werden.

## Patentansprüche

1. Doppelsträngige Interferenz-RNA von 21 Nukleotiden Länge, die mindestens eine chemische Modifikation aufweist, wobei die doppelsträngige Interferenz-RNA durch Ersetzen, Anfügen oder Entfernen einzelner oder mehrerer Atome oder Atomgruppen im Vergleich zu einer natürlich vorkommenden RNA-Spezies verändert ist, zur Verwendung als immunstimulierendes Mittel.

2. Doppelsträngige Interferenz-RNA zur Verwendung nach Anspruch 1, **dadurch gekennzeichnet, dass** mindestens ein Nucleotid der RNA ein Analoges natürlich vorkommender Nucleotide ist.

3. Doppelsträngige Interferenz-RNA zur Verwendung nach Anspruch 2, **dadurch gekennzeichnet, dass** die RNA aus Nucleotidanaloga besteht.

4. Doppelsträngige Interferenz-RNA zur Verwendung nach Anspruch 2 oder 3, **dadurch gekennzeichnet, dass** das Analoge aus der Gruppe, bestehend aus Phosphorthioaten, Phosphoramidaten, Peptidnucleotiden, Methylphosphonaten, 7-Deazaguanosin, 5-Methylcytosin und Inosin, ausgewählt ist.

5. Doppelsträngige Interferenz-RNA zur Verwendung nach einem der Ansprüche 1 bis 4, **dadurch gekennzeichnet, dass** das Mittel mindestens ein Adjuvans enthält.

6. Doppelsträngige Interferenz-RNA zur Verwendung nach Anspruch 5, **dadurch gekennzeichnet, dass** das Adjuvans ausgewählt wird aus der Gruppe, bestehend aus Cytokinen, umfassend Monokine, Lymphokine, Interleukine, Chemokine, IL-1, IL-2, IL-3, IL-4, IL-5, IL-6, IL-7, IL-8, IL-9, IL-10, IL-12, IFN-α, IFN-γ, GM-CSF, LT-α, Lipopeptiden, CpG-Oligonucleotiden, CpG-DNA, Lipopolysacchariden, TNF-α, CD40-Liganden, Gefahrsignalen ausgewählt aus LPS oder GP96, Wachstumsfaktoren, umfassend hGH, Aluminiumhydroxid, Freund'sches Adjuvans, kationischen Peptiden oder Polypeptiden, umfassend Protamin, Poly-L-Lysin, Histone, kationischen Polysacchariden, umfassend Chitosan, Lipopeptiden, und Pam3Cys.

7. Doppelsträngige Interferenz-RNA zur Verwendung nach einem der Ansprüche 1 bis 6, **dadurch gekennzeichnet, dass** das Mittel weiter einen pharmazeutisch verträglichen Träger und/oder ein pharmazeutisch verträgliches Vehikel enthält.

8. Doppelsträngige Interferenz-RNA nach einem der Ansprüche 1 bis 7, zur Verwendung in der Prävention und/oder Behandlung von Infektionserkrankungen oder Krebserkrankungen.

9. Verwendung einer doppelsträngigen Interferenz-RNA von 21 Nukleotiden Länge, die mindestens eine chemische Modifikation aufweist, zur Herstellung eines immunstimulierenden Mittels.

10. Vakzine, enthaltend mindestens eine doppelsträngige Interferenz-RNA von 21 Nukleotiden Länge, die mindestens eine chemische Modifikation aufweist, wobei die doppelsträngige Interferenz-RNA durch Ersetzen, Anfügen oder Entfernen einzelner oder mehrerer Atome oder Atomgruppen im Vergleich zu einer natürlich vorkommenden RNA-Spezies verändert ist, und mindestens ein Antigen.

11. Vakzine nach Anspruch 10, **dadurch gekennzeichnet, dass** das Antigen aus der Gruppe, bestehend aus Peptiden, Polypeptiden, Zellen, Zellextrakten, Polysacchariden, Polysaccharidkonjugaten, Lipiden, Glykolipiden und Kohlenhydraten, ausgewählt ist, wobei das Peptid- oder Polypeptidantigen bevorzugt in Form einer dafür codierenden Nucleinsäure vorliegt oder die Nucleinsäure bevorzugt eine mRNA ist, stärker bevorzugt eine stabilisierte und/oder translationsoptimierte mRNA ist.

12. Vakzine nach Anspruch 10 oder 11, **dadurch gekennzeichnet, dass** das Antigen aus Tumorantigenen und Antigenen von Viren, Bakterien, Pilzen und Protozoen ausgewählt ist, wobei das virale, bakterielle, Pilz- oder protozoologische Antigen bevorzugt aus einem sekretierten Protein stammt oder das Antigen bevorzugt ein Polyepitop von Tumorantigenen oder Antigen von Viren, Bakterien, Pilzen oder Protozoen ist.

13. Vakzine nach einem der Ansprüche 10 bis 12 zur Verwendung der Impfung gegen Infektionserkrankungen oder Krebserkrankungen.

## Claims

1. Double-stranded interference-RNA having a length of 21 nucleotides, which has at least one chemical modification, wherein the double-stranded interference-RNA is modified by substitution, addition or deletion of single or several atoms or atom groups in comparison to a naturally occurring RNA-species, for use as an immunostimulatory agent.

2. Double-stranded interference-RNA for use according to claim 1, **characterized in that** at least one nucleotide of the RNA is an analogue of naturally occurring nucleotides.

3. Double-stranded interference-RNA for use according to claim 2, **characterized in that** the RNA consists of nucleotide analogues.

4. Double-stranded interference-RNA for use according to 2 or 3, **characterized in that** the analogue is selected from the group consisting of phosphorothioates, phosphoroamidates, peptide nucleotides, methylphosphonates, 7-deazaguanosine, 5-methylcytosine and inosine.

5. Double-stranded interference-RNA for use according to any of claims 1 to 4, **characterized in that** the agent comprises at least one adjuvant.

6. Double-stranded interference-RNA for use according to claim 5, **characterized in that** the adjuvant is selected from the group, consisting of cytokines, comprising monokines, lymphokines, interleukines, chemokines, IL-1, IL-2, IL-3, IL-4, IL-5, IL-6, IL-7, IL-8, IL-9, IL-10, IL-12, IFN-α, IFN-γ, GM-CSF, Lot-α, lipopeptides, CpG-oligonucleotides, CpG-DNA, lipopolysaccharides, TNF-α, CD40-ligands, danger signals selected from LPS or GP96, growth factors comprising hGH, aluminium hydroxide, Freunds' adjuvant, cationic peptides or polypeptides comprising protamin, poly-L-lysine, histones, cationic polysaccharides comprising chitosan, lipopeptides, and Pam3Cys.

7. Double-stranded interference-RNA for use according to any of claims 1 to 6, **characterized in that** the agent furthermore comprises a pharmaceutically acceptable carrier and/or a pharmaceutically acceptable vehicle.

8. Double-stranded interference-RNA according to any of claims 1 to 7 for use in the prevention and/or treatment of infectious diseases or cancer diseases.

9. Use of a double-stranded interference-RNA having a length of 21 nucleotides, which has at least one chemical modification, for the manufacture of an immunostimulatory agent.

10. Vaccine comprising at least one double-stranded interference-RNA having a length of 21 nucleotides, which has at least one chemical modification, wherein the double-stranded interference-RNA is modified by substitution, addition or deletion of single or more atoms or atom groups in comparison to a naturally occurring RNA-species, and at least one antigen.

11. Vaccine according to claim 10, **characterized in that** the antigen is selected from the group consisting of peptides, polypeptides, cells, cell extracts, polysaccharides, polysaccharide conjugates, lipids, glycolipids and carbohydrates, wherein the peptide or polypeptide antigen is preferably present in the form of a nucleic acid encoding the same, or wherein the nucleic acid preferably is an mRNA, more preferably stabilized and/or translation-optimized mRNA.

12. Vaccine according to claim 10 or 11, **characterized in that** the antigen is selected from tumor antigens and antigens of viruses, bacteria, fungi and protozoa, wherein the viral, bacterial, fungal or protozoological antigen is preferably derived from a secreted protein or wherein the antigen is preferably a polyepitope of tumor antigens or antigens of viruses, bacteria, fungi or protozoa.

13. Vaccine according to any of claims 10 to 12 for use in vaccination against infectious diseases or cancer diseases.

## Revendications

1. ARN interférent double brin ayant une longueur de 21 nucléotides, qui présente au moins une modification chimique, l'ARN interférent double brin étant modifié par substitution, addition ou délétion d'un ou plusieurs atomes ou groupes d'atomes, par comparaison avec une espèce d'ARN naturelle, pour utilisation en tant qu'agent immunostimulant.

2. ARN interférent double brin pour l'utilisation selon la revendication 1, **caractérisé en ce qu'**au moins un nucléotide de l'ARN est un analogue de nucléotides naturels.

3. ARN interférent double brin pour l'utilisation selon la revendication 2, **caractérisé en ce que** l'ARN est constitué d'analogues de nucléotides.

4. ARN interférent double brin pour l'utilisation selon la revendication 2 ou 3, **caractérisé en ce que** l'analogue est choisi dans le groupe consistant en les phosphorothioates, les phosphoramidates, les nucléotides peptidiques, les méthylphosphonates, la 7-déazaguanosine, la 5-méthylcytosine et l'inosine.

5. ARN interférent double brin pour l'utilisation selon l'une des revendications 1 à 4, **caractérisé en ce que** l'agent contient au moins un adjuvant.

6. ARN interférent double brin pour l'utilisation selon la revendication 5, **caractérisé en ce que** l'adjuvant est choisi dans le groupe consistant en les cytokines, comprenant les monokines, les lymphokines, les interleukines, les chimiokines, l'IL-1, l'IL-2, l'IL-3, l'IL-4, l'IL-5, l'IL-6, l'IL-7, l'IL-8, l'IL-9, l'IL-10, l'IL-12, l'IFN-,α l'IFN-γ, le GM-CSF, le LT-α, les lipopeptides, les oligonucléotides CpG, le CpG-ADN, les lipopolysaccharides, le TNF-α, les ligands CD40, les signaux de danger choisis parmi les LPS ou le GP96, les facteurs de croissance, comprenant le hGH, l'hydroxyde d'aluminium, l'adjuvant de Freund, les peptides ou polypeptides cationiques, comprenant la protamine, la poly-L-lysine, les histones, les polysaccharides cationiques, comprenant le chitosan, les lipopeptides et le Pam3Cys.

7. ARN interférent double brin pour l'utilisation selon l'une des revendications 1 à 6, **caractérisé en ce que** l'agent contient en outre un support pharmaceutiquement compatible et/ou un véhicule pharmaceutiquement compatible.

8. ARN interférent double brin selon l'une des revendications 1 à 7 pour l'utilisation pour la prévention et/ou le traitement de maladies infectieuses ou de maladies cancéreuses.

9. Utilisation d'un ARN interférent double brin ayant une longueur de 21 nucléotides, qui comprend au moins une modification chimique, pour préparation d'un agent immunostimulant.

10. Vaccins contenant au moins un ARN interférent double brin ayant une longueur de 21 nucléotides, qui comprend au moins une modification chimique, l'ARN interférent double brin étant modifié par substitution, addition ou délétion d'un ou plusieurs atomes ou groupes d'atomes, par comparaison avec une espèce d'ARN naturelle, et au moins un antigène.

11. Vaccins selon la revendication 10, **caractérisés en ce que** l'antigène est choisi dans le groupe consistant en les peptides, les polypeptides, les cellules, les extraits cellulaires, les polysaccharides, les conjugués polysaccharidiques, les lipides, les glycolipides et les hydrates de carbone, l'antigène peptidique ou polypeptidique se présentant de préférence sous forme d'un acide nucléique codant pour eux, ou l'acide nucléique étant de préférence un ARNm, plus préférentiellement un ARNm stabilisé et/ou optimisé en traduction.

12. Vaccins selon la revendication 10 ou 11, **caractérisés en ce que** l'antigène est choisi parmi les antigènes tumoraux et les antigènes de virus, de bactéries, de champignons et de protozoaires, l'antigène viral, bactérien, fongique ou protozoaire provenant de préférence d'une protéine sécrétée, ou l'antigène est de préférence un polyépitope d'antigènes tumoraux ou un antigène de virus, de bactéries, de champignons ou de protozoaires.

13. Vaccins selon l'une des revendications 10 à 12 pour utilisation lors d'une vaccination contre des maladies infectieuses ou des maladies cancéreuses.


### SEQUENCE LISTING

<110> curevac GmbH
<120> Immunstimulation durch chemisch modifizierte RNA
<130> CU01P013WOEPT1T2
<140>
   <141>
<150> EP 06007325.1
   <151> 2003-07-03
<150> WO2004004743
   <151> 2003-07-03
<160> 8
<170> PatentIn version 3.3
<210> 1
   <211> 13
   <212> RNA
   <213> mammalian
<220>
   <221> misc_feature
   <223> Kozak-sequenz (siehe Beschreibung Seite 1)
<400> 1
   gccgccacca ugg 13
<210> 2
   <211> 20
   <212> RNA
   <213> Artificial
<220>
   <223> Beschreibung der Sequenz: CpG RNA 1668 (siehe Beschreibung Seite 6)
<400> 2
   uccaugacgu uccugaugcu 20
<210> 3
   <211> 20
   <212> RNA
   <213> Artificial
<220>
   <223> Beschreibung der Sequenz: CpG RNA 1826 (siehe Beschreibung Seite 6)
<400> 3
   uccaugacgu uccugacguu 20
<210> 4
   <211> 20
   <212> RNA
   <213> Artificial
<220>
   <223> Beschreibung der Sequenz: RNA 1982 (siehe Beschreibung Seite 6)
<400> 4
   uccaggacuu cucucagguu 20
<210> 5
   <211> 15
   <212> RNA
   <213> Artificial
<220>
   <223> Beschreibung der Sequenz: generic stabilizing sequence of the formula (C/U)CCANxCCC(U/A)PyxUC(C/U)CC
<220>
   <221> variation
   <222> (1)..(1)
   <223> /replace="cytosine" /replace="uracile"
<220>
   <221> variation
   <222> (5)..(5)
   <223> /replace="cytosine" /replace="uracile" /replace="guanosine" /replace="adenosine"
<220>
   <221> repeat_unit
   <222> (5)..(5)
   <223> x = any number
<220>
   <221> variation
   <222> (9)..(9)
   <223> /replace="uracile" /replace="adonosine"
<220>
   <221> repeat_unit
   <222> (10)..(10)
   <223> x = any number
<220>
   <221> variation
   <222> (10)..(10)
   <223> /replace="pyrimidine"
<220>
   <221> variation
   <222> (13)..(13)
   <223> /replace="cytosine" /replace="uracile"
<400> 5
   nccancccnn ucncc 15
<210> 6
   <211> 20
   <212> DNA
   <213> Artificial
<220>
   <223> Beschreibung der Sequenz: CpG DNA 1668 (siehe Beschreibung Seite 36)
<400> 6
   tccatgacgt tcctgatgct 20
<210> 7
   <211> 20
   <212> DNA
   <213> Artificial
<220>
   <223> Beschreibung der Sequenz: CpG DNA 1826 (siehe Beschreibung Seite 36)
<400> 7
   tccatgacgt tcctgacgtt 20
<210> 8
   <211> 20
   <212> DNA
   <213> Artificial
<220>
   <223> Beschreibung der Sequenz: DNA 1982 (siehe Beschreibung Seite 36)
<400> 8
   tccaggactt ctctcaggtt 20